(19) Europäisches Patentamt
European Patent Office
Office européen des brevets

(11) EP 3 184 544 A1

(12) **EUROPEAN PATENT APPLICATION**

(43) Date of publication:
**28.06.2017 Bulletin 2017/26**

(21) Application number: **15202582.1**

(22) Date of filing: **23.12.2015**

(51) Int Cl.:
***C07K 16/28*** *(2006.01)* ***C07K 16/36*** *(2006.01)*
***A61P 7/04*** *(2006.01)* ***A61K 39/395*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Julius-Maximilians-Universität Würzburg**
**97070 Würzburg (DE)**

(72) Inventor: **The designation of the inventor has not yet been filed**

(74) Representative: **Kalhammer, Georg et al**
**Lederer & Keller**
**Patentanwälte Partnerschaft mbB**
**Unsöldstrasse 2**
**80538 München (DE)**

(54) **GLYCOPROTEIN V INHIBITORS FOR USE AS COAGULANTS**

(57) The present invention relates to an inhibitor of glycoprotein V (GPV) for use as a coagulant, and/or for use in the treatment or prevention of a hemorrhagic condition.

EP 3 184 544 A1

SEQUENCE LISTING

```
<110>  Julius-Maximilians-Universitaet Wuerzburg

<120>  Glycoprotein V inhibitors for use as coagulants

<130>  2015_M003_A252

<160>  6

<170>  PatentIn version 3.5

<210>  1
<211>  3493
<212>  DNA
<213>  Homo sapiens


<220>
<221>  CDS
<222>  (32)..(1714)

<400>  1
agttactttg gagtgcagaa ccatttcaga c atg ctg agg ggg act cta ctg           52
                                   Met Leu Arg Gly Thr Leu Leu
                                   1               5

tgc gcg gtg ctc ggg ctt ctg cgc gcc cag ccc ttc ccc tgt ccg cca         100
Cys Ala Val Leu Gly Leu Leu Arg Ala Gln Pro Phe Pro Cys Pro Pro
        10                  15                  20

gct tgc aag tgt gtc ttc cgg gac gcc gcg cag tgc tcg ggg ggc gac         148
Ala Cys Lys Cys Val Phe Arg Asp Ala Ala Gln Cys Ser Gly Gly Asp
    25                  30                  35

gtg gcg cgc atc tcc gcg cta ggc ctg ccc acc aac ctc acg cac atc         196
Val Ala Arg Ile Ser Ala Leu Gly Leu Pro Thr Asn Leu Thr His Ile
40                  45                  50                  55

ctg ctc ttc gga atg ggc cgc ggc gtc ctg cag agc cag agc ttc agc         244
Leu Leu Phe Gly Met Gly Arg Gly Val Leu Gln Ser Gln Ser Phe Ser
                60                  65                  70

ggc atg acc gtc ctg cag cgc ctc atg atc tcc gac agc cac att tcc         292
Gly Met Thr Val Leu Gln Arg Leu Met Ile Ser Asp Ser His Ile Ser
            75                  80                  85

gcc gtt gcc ccc ggc acc ttc agt gac ctg ata aaa ctg aaa acc ctg         340
Ala Val Ala Pro Gly Thr Phe Ser Asp Leu Ile Lys Leu Lys Thr Leu
        90                  95                  100

agg ctg tcg cgc aac aaa atc acg cat ctt cca ggt gcg ctg ctg gat         388
Arg Leu Ser Arg Asn Lys Ile Thr His Leu Pro Gly Ala Leu Leu Asp
    105                 110                 115

aag atg gtg ctc ctg gag cag ttg ttt ttg gac cac aat gcg cta agg         436
Lys Met Val Leu Leu Glu Gln Leu Phe Leu Asp His Asn Ala Leu Arg
120                 125                 130                 135

ggc att gac caa aac atg ttt cag aaa ctg gtt aac ctg cag gag ctc         484
Gly Ile Asp Gln Asn Met Phe Gln Lys Leu Val Asn Leu Gln Glu Leu
                140                 145                 150
```

```
gct ctg aac cag aat cag ctc gat ttc ctt cct gcc agt ctc ttc acg        532
Ala Leu Asn Gln Asn Gln Leu Asp Phe Leu Pro Ala Ser Leu Phe Thr
            155                 160                 165

aat ctg gag aac ctg aag ttg ttg gat tta tcg gga aac aac ctg acc        580
Asn Leu Glu Asn Leu Lys Leu Leu Asp Leu Ser Gly Asn Asn Leu Thr
        170                 175                 180

cac ctg ccc aag ggg ttg ctt gga gca cag gct aag ctc gag aga ctt        628
His Leu Pro Lys Gly Leu Leu Gly Ala Gln Ala Lys Leu Glu Arg Leu
    185                 190                 195

ctg ctc cac tcg aac cgc ctt gtg tct ctg gat tcg ggg ctg ttg aac        676
Leu Leu His Ser Asn Arg Leu Val Ser Leu Asp Ser Gly Leu Leu Asn
200                 205                 210                 215

agc ctg ggc gcc ctg acg gag ctg cag ttc cac cga aat cac atc cgt        724
Ser Leu Gly Ala Leu Thr Glu Leu Gln Phe His Arg Asn His Ile Arg
                220                 225                 230

tcc atc gca ccc ggg gcc ttc gac cgg ctc cca aac ctc agt tct ttg        772
Ser Ile Ala Pro Gly Ala Phe Asp Arg Leu Pro Asn Leu Ser Ser Leu
            235                 240                 245

acg ctt tcg aga aac cac ctt gcg ttt ctc ccc tct gcg ctc ttt ctt        820
Thr Leu Ser Arg Asn His Leu Ala Phe Leu Pro Ser Ala Leu Phe Leu
        250                 255                 260

cat tcg cac aat ctg act ctg ttg act ctg ttc gag aac ccg ctg gca        868
His Ser His Asn Leu Thr Leu Leu Thr Leu Phe Glu Asn Pro Leu Ala
    265                 270                 275

gag ctc ccg ggg gtg ctc ttc ggg gag atg ggg ggc ctg cag gag ctg        916
Glu Leu Pro Gly Val Leu Phe Gly Glu Met Gly Gly Leu Gln Glu Leu
280                 285                 290                 295

tgg ctg aac cgc acc cag ctg cgc acc ctg ccc gcc gcc gcc ttc cgc        964
Trp Leu Asn Arg Thr Gln Leu Arg Thr Leu Pro Ala Ala Ala Phe Arg
                300                 305                 310

aac ctg agc cgc ctg cgg tac tta ggg gtg act ctg agc ccg cgg ctg       1012
Asn Leu Ser Arg Leu Arg Tyr Leu Gly Val Thr Leu Ser Pro Arg Leu
            315                 320                 325

agc gcg ctt ccg cag ggc gcc ttc cag ggc ctt ggc gag ctc cag gtg       1060
Ser Ala Leu Pro Gln Gly Ala Phe Gln Gly Leu Gly Glu Leu Gln Val
        330                 335                 340

ctc gcc ctg cac tcc aac ggc ctg acc gcc ctc ccc gac ggc ttg ctg       1108
Leu Ala Leu His Ser Asn Gly Leu Thr Ala Leu Pro Asp Gly Leu Leu
    345                 350                 355

cgc ggc ctc ggc aag ctg cgc cag gtg tcc ctg cgc cgc aac agg ctg       1156
Arg Gly Leu Gly Lys Leu Arg Gln Val Ser Leu Arg Arg Asn Arg Leu
360                 365                 370                 375

cgc gcc ctg ccc cgt gcc ctc ttc cgc aat ctc agc agc ctg gag agc       1204
Arg Ala Leu Pro Arg Ala Leu Phe Arg Asn Leu Ser Ser Leu Glu Ser
                380                 385                 390

gtc cag ctc gac cac aac cag ctg gag acc ctg cct ggc gac gtg ttt       1252
Val Gln Leu Asp His Asn Gln Leu Glu Thr Leu Pro Gly Asp Val Phe
            395                 400                 405
```

```
ggg gct ctg ccc cgg ctg acg gag gtc ctg ttg ggg cac aac tcc tgg        1300
Gly Ala Leu Pro Arg Leu Thr Glu Val Leu Leu Gly His Asn Ser Trp
        410                 415                 420

cgc tgc gac tgt ggc ctg ggg ccc ttc ctg ggg tgg ctg cgg cag cac        1348
Arg Cys Asp Cys Gly Leu Gly Pro Phe Leu Gly Trp Leu Arg Gln His
    425                 430                 435

cta ggc ctc gtg ggc ggg gaa gag ccc cca cgg tgc gca ggc cct ggg        1396
Leu Gly Leu Val Gly Gly Glu Glu Pro Pro Arg Cys Ala Gly Pro Gly
440                 445                 450                 455

gcg cac gcc ggc ctg ccg ctc tgg gcc ctg ccg ggg ggt gac gcg gag        1444
Ala His Ala Gly Leu Pro Leu Trp Ala Leu Pro Gly Gly Asp Ala Glu
                460                 465                 470

tgc ccg ggc ccc cgg ggc ccg cct ccc cgc ccc gct gcg gac agc tcc        1492
Cys Pro Gly Pro Arg Gly Pro Pro Pro Arg Pro Ala Ala Asp Ser Ser
            475                 480                 485

tcg gaa gcc cct gtc cac cca gcc ttg gct ccc aac agc tca gaa ccc        1540
Ser Glu Ala Pro Val His Pro Ala Leu Ala Pro Asn Ser Ser Glu Pro
        490                 495                 500

tgg gtg tgg gcc cag ccg gtg acc acg ggc aaa ggt caa gat cat agt        1588
Trp Val Trp Ala Gln Pro Val Thr Thr Gly Lys Gly Gln Asp His Ser
    505                 510                 515

ccg ttc tgg ggg ttt tat ttt ctg ctt tta gct gtt cag gcc atg atc        1636
Pro Phe Trp Gly Phe Tyr Phe Leu Leu Leu Ala Val Gln Ala Met Ile
520                 525                 530                 535

acc gtg atc atc gtg ttt gct atg att aaa att ggc caa ctc ttt cga        1684
Thr Val Ile Ile Val Phe Ala Met Ile Lys Ile Gly Gln Leu Phe Arg
                540                 545                 550

aaa tta atc aga gag aga gcc ctt ggg taa accaatggga aaatcttcta          1734
Lys Leu Ile Arg Glu Arg Ala Leu Gly
            555                 560

attacttaga acctgaccag atgtggctcg gaggggaatc cagacccgct gctgtcttgc      1794

tctccctccc ctccccactc ctcctctctt cttcctcttc tctctcactg ccacgccttc      1854

ctttccctcc tcctccccct ctccgctctg tgctcttcat tctcacaggc ccgcaacccc      1914

tcctctctgt gtcccccgcc cgttcctgga aactgagctt gacgtttgta aactgtggtt      1974

gcctgccttc cccagctccc acgcgggtgt gcgctgacac tgccgggggc gctggactgt      2034

gttggaccca tccgtgctcc gctgtgcctg gcttggcgtc tggtggagag aggggcctct      2094

tcagtgtcta ctgagtaagg ggacagctcc aggccggggc ctgtctcctg cacagagtaa      2154

gccggtaaat gtttgtgaaa tcaatgcgtg gataaaggaa cacatgccat ccaagtgatg      2214

atggcttttc ctggagggaa aggataggct gttgctctat ctaatttttt gtttttgttt      2274

ttggacagtc tagctctgtg gcccaggctg gcgtgcagtg ggccgtctca gttcactgca      2334

gcctccgcct cccaggttca agtgattctc atgcctcagc gttctgagta gctgggatta      2394
```

gaggcgtgtg ccactacacc cggctaattt ttgtactttt taaagtagag acggggcttt 2454

gccatattgg cctggctgat ctcaaactcc tggtcttgaa ctcctggcca caagtgatct 2514

gcccgccttg gcctcccaaa gtgctgggat tacaggcgta agccactaca cctggccctc 2574

ttcatcgaat tttatttgag aagtagagct cttgccattt tttcccttgc tccatttttc 2634

tcactttatg tctctctgac ctatgggcta cttgggagag cactggactc cattcatgca 2694

tgagcatttt caggataagc gacttctgtg aggctgagag aggaagaaaa cacggagcct 2754

tccctccagg tgcccagtgt aggtccagcg tgtttcctga gcctcctgtg agtttccact 2814

tgctttacat ccatgcaaca tgtcattttg aaactggatt gatttgcatt tcctggaact 2874

ctgccacctc atttcacaag catttatgga gcagttaaca tgtgactggt attcatgaat 2934

ataatgataa gcttgattct agttcagctg ctgtcacagt ctcatttgtt cttccaactg 2994

aaagccgtaa aacctttgtt gctttaattg aatgtctgtg cttatgagag gcagtggtta 3054

aaacaggggc tggcgagttg acaactgtgg gttcaaatcc cagctctacc acttactaac 3114

tgcatgggac tttgggtaag acacctgctt acattctcta agccttggtt tcctgaacct 3174

taaaacagga taacatagta cctgcttcgt agagtttttg tgagaattaa aggcaataaa 3234

gcatataatg acttagccca gcggcctgca ggcaatacat gttaatgaat gttagctatt 3294

attactaaag gatgagcaat tattattggc atcatgattt ctaaagaaga gctttgagtt 3354

ggtatttttc tctgtgtata agggtaagtc cgaactttct cagactggag gttacattca 3414

catcagtctg tcttcccctg cggatggcct cagccctggg tggccagact ctgtgctcac 3474

aatccagagc aatggatcc 3493

<210> 2
<211> 560
<212> PRT
<213> Homo sapiens

<400> 2

Met Leu Arg Gly Thr Leu Leu Cys Ala Val Leu Gly Leu Leu Arg Ala
1 5 10 15

Gln Pro Phe Pro Cys Pro Pro Ala Cys Lys Cys Val Phe Arg Asp Ala
20 25 30

Ala Gln Cys Ser Gly Gly Asp Val Ala Arg Ile Ser Ala Leu Gly Leu
35 40 45

Pro Thr Asn Leu Thr His Ile Leu Leu Phe Gly Met Gly Arg Gly Val
50 55 60

Leu Gln Ser Gln Ser Phe Ser Gly Met Thr Val Leu Gln Arg Leu Met

```
65                  70                  75                  80

Ile Ser Asp Ser His Ile Ser Ala Val Ala Pro Gly Thr Phe Ser Asp
                85                  90                  95

Leu Ile Lys Leu Lys Thr Leu Arg Leu Ser Arg Asn Lys Ile Thr His
            100                 105                 110

Leu Pro Gly Ala Leu Leu Asp Lys Met Val Leu Leu Glu Gln Leu Phe
        115                 120                 125

Leu Asp His Asn Ala Leu Arg Gly Ile Asp Gln Asn Met Phe Gln Lys
    130                 135                 140

Leu Val Asn Leu Gln Glu Leu Ala Leu Asn Gln Asn Gln Leu Asp Phe
145                 150                 155                 160

Leu Pro Ala Ser Leu Phe Thr Asn Leu Glu Asn Leu Lys Leu Leu Asp
                165                 170                 175

Leu Ser Gly Asn Asn Leu Thr His Leu Pro Lys Gly Leu Leu Gly Ala
            180                 185                 190

Gln Ala Lys Leu Glu Arg Leu Leu Leu His Ser Asn Arg Leu Val Ser
        195                 200                 205

Leu Asp Ser Gly Leu Leu Asn Ser Leu Gly Ala Leu Thr Glu Leu Gln
    210                 215                 220

Phe His Arg Asn His Ile Arg Ser Ile Ala Pro Gly Ala Phe Asp Arg
225                 230                 235                 240

Leu Pro Asn Leu Ser Ser Leu Thr Leu Ser Arg Asn His Leu Ala Phe
                245                 250                 255

Leu Pro Ser Ala Leu Phe Leu His Ser His Asn Leu Thr Leu Leu Thr
            260                 265                 270

Leu Phe Glu Asn Pro Leu Ala Glu Leu Pro Gly Val Leu Phe Gly Glu
        275                 280                 285

Met Gly Gly Leu Gln Glu Leu Trp Leu Asn Arg Thr Gln Leu Arg Thr
    290                 295                 300

Leu Pro Ala Ala Ala Phe Arg Asn Leu Ser Arg Leu Arg Tyr Leu Gly
305                 310                 315                 320
```

```
Val Thr Leu Ser Pro Arg Leu Ser Ala Leu Pro Gln Gly Ala Phe Gln
                325                 330                 335

Gly Leu Gly Glu Leu Gln Val Leu Ala Leu His Ser Asn Gly Leu Thr
            340                 345                 350

Ala Leu Pro Asp Gly Leu Leu Arg Gly Leu Gly Lys Leu Arg Gln Val
        355                 360                 365

Ser Leu Arg Arg Asn Arg Leu Arg Ala Leu Pro Arg Ala Leu Phe Arg
    370                 375                 380

Asn Leu Ser Ser Leu Glu Ser Val Gln Leu Asp His Asn Gln Leu Glu
385                 390                 395                 400

Thr Leu Pro Gly Asp Val Phe Gly Ala Leu Pro Arg Leu Thr Glu Val
                405                 410                 415

Leu Leu Gly His Asn Ser Trp Arg Cys Asp Cys Gly Leu Gly Pro Phe
            420                 425                 430

Leu Gly Trp Leu Arg Gln His Leu Gly Leu Val Gly Gly Glu Glu Pro
        435                 440                 445

Pro Arg Cys Ala Gly Pro Gly Ala His Ala Gly Leu Pro Leu Trp Ala
    450                 455                 460

Leu Pro Gly Gly Asp Ala Glu Cys Pro Gly Pro Arg Gly Pro Pro Pro
465                 470                 475                 480

Arg Pro Ala Ala Asp Ser Ser Ser Glu Ala Pro Val His Pro Ala Leu
                485                 490                 495

Ala Pro Asn Ser Ser Glu Pro Trp Val Trp Ala Gln Pro Val Thr Thr
            500                 505                 510

Gly Lys Gly Gln Asp His Ser Pro Phe Trp Gly Phe Tyr Phe Leu Leu
        515                 520                 525

Leu Ala Val Gln Ala Met Ile Thr Val Ile Ile Val Phe Ala Met Ile
    530                 535                 540

Lys Ile Gly Gln Leu Phe Arg Lys Leu Ile Arg Glu Arg Ala Leu Gly
545                 550                 555                 560
```

<210> 3
<211> 544
<212> PRT

<213> Homo sapiens

<400> 3

```
Gln Pro Phe Pro Cys Pro Pro Ala Cys Lys Cys Val Phe Arg Asp Ala
1               5                   10                  15

Ala Gln Cys Ser Gly Gly Asp Val Ala Arg Ile Ser Ala Leu Gly Leu
            20                  25                  30

Pro Thr Asn Leu Thr His Ile Leu Leu Phe Gly Met Gly Arg Gly Val
        35                  40                  45

Leu Gln Ser Gln Ser Phe Ser Gly Met Thr Val Leu Gln Arg Leu Met
    50                  55                  60

Ile Ser Asp Ser His Ile Ser Ala Val Ala Pro Gly Thr Phe Ser Asp
65                  70                  75                  80

Leu Ile Lys Leu Lys Thr Leu Arg Leu Ser Arg Asn Lys Ile Thr His
                85                  90                  95

Leu Pro Gly Ala Leu Leu Asp Lys Met Val Leu Leu Glu Gln Leu Phe
            100                 105                 110

Leu Asp His Asn Ala Leu Arg Gly Ile Asp Gln Asn Met Phe Gln Lys
        115                 120                 125

Leu Val Asn Leu Gln Glu Leu Ala Leu Asn Gln Asn Gln Leu Asp Phe
    130                 135                 140

Leu Pro Ala Ser Leu Phe Thr Asn Leu Glu Asn Leu Lys Leu Leu Asp
145                 150                 155                 160

Leu Ser Gly Asn Asn Leu Thr His Leu Pro Lys Gly Leu Leu Gly Ala
                165                 170                 175

Gln Ala Lys Leu Glu Arg Leu Leu Leu His Ser Asn Arg Leu Val Ser
            180                 185                 190

Leu Asp Ser Gly Leu Leu Asn Ser Leu Gly Ala Leu Thr Glu Leu Gln
        195                 200                 205

Phe His Arg Asn His Ile Arg Ser Ile Ala Pro Gly Ala Phe Asp Arg
    210                 215                 220

Leu Pro Asn Leu Ser Ser Leu Thr Leu Ser Arg Asn His Leu Ala Phe
225                 230                 235                 240
```

```
Leu Pro Ser Ala Leu Phe Leu His Ser His Asn Leu Thr Leu Leu Thr
                245                 250                 255

Leu Phe Glu Asn Pro Leu Ala Glu Leu Pro Gly Val Leu Phe Gly Glu
            260                 265                 270

Met Gly Gly Leu Gln Glu Leu Trp Leu Asn Arg Thr Gln Leu Arg Thr
        275                 280                 285

Leu Pro Ala Ala Ala Phe Arg Asn Leu Ser Arg Leu Arg Tyr Leu Gly
    290                 295                 300

Val Thr Leu Ser Pro Arg Leu Ser Ala Leu Pro Gln Gly Ala Phe Gln
305                 310                 315                 320

Gly Leu Gly Glu Leu Gln Val Leu Ala Leu His Ser Asn Gly Leu Thr
                325                 330                 335

Ala Leu Pro Asp Gly Leu Leu Arg Gly Leu Gly Lys Leu Arg Gln Val
            340                 345                 350

Ser Leu Arg Arg Asn Arg Leu Arg Ala Leu Pro Arg Ala Leu Phe Arg
        355                 360                 365

Asn Leu Ser Ser Leu Glu Ser Val Gln Leu Asp His Asn Gln Leu Glu
    370                 375                 380

Thr Leu Pro Gly Asp Val Phe Gly Ala Leu Pro Arg Leu Thr Glu Val
385                 390                 395                 400

Leu Leu Gly His Asn Ser Trp Arg Cys Asp Cys Gly Leu Gly Pro Phe
                405                 410                 415

Leu Gly Trp Leu Arg Gln His Leu Gly Leu Val Gly Gly Glu Glu Pro
            420                 425                 430

Pro Arg Cys Ala Gly Pro Gly Ala His Ala Gly Leu Pro Leu Trp Ala
        435                 440                 445

Leu Pro Gly Gly Asp Ala Glu Cys Pro Gly Pro Arg Gly Pro Pro Pro
    450                 455                 460

Arg Pro Ala Ala Asp Ser Ser Ser Glu Ala Pro Val His Pro Ala Leu
465                 470                 475                 480

Ala Pro Asn Ser Ser Glu Pro Trp Val Trp Ala Gln Pro Val Thr Thr
                485                 490                 495
```

```
Gly Lys Gly Gln Asp His Ser Pro Phe Trp Gly Phe Tyr Phe Leu Leu
            500                 505                 510

Leu Ala Val Gln Ala Met Ile Thr Val Ile Ile Val Phe Ala Met Ile
        515                 520                 525

Lys Ile Gly Gln Leu Phe Arg Lys Leu Ile Arg Glu Arg Ala Leu Gly
    530                 535                 540


<210>  4
<211>  2215
<212>  DNA
<213>  Mus musculus


<220>
<221>  CDS
<222>  (30)..(1733)

<400>  4
tcagtccagg gtgcagaact gcttcagac atg cta aga agc gcc ctg ctg tcc        53
                                Met Leu Arg Ser Ala Leu Leu Ser
                                1               5

gcg gtg ctc gca ctc ttg cgt gcc caa cct ttt ccc tgc ccc aaa acc       101
Ala Val Leu Ala Leu Leu Arg Ala Gln Pro Phe Pro Cys Pro Lys Thr
    10                  15                  20

tgc aag tgt gtg gtc cgc gat gcc gcg cag tgc tcg ggc ggc agc gtg       149
Cys Lys Cys Val Val Arg Asp Ala Ala Gln Cys Ser Gly Gly Ser Val
25                  30                  35                  40

gct cac atc gct gag cta ggt ctg cct acg aac ctc aca cac atc ctg       197
Ala His Ile Ala Glu Leu Gly Leu Pro Thr Asn Leu Thr His Ile Leu
                45                  50                  55

ctc ttc cga atg gac cag ggc ata ttg cgg aac cac agc ttc agc ggc       245
Leu Phe Arg Met Asp Gln Gly Ile Leu Arg Asn His Ser Phe Ser Gly
            60                  65                  70

atg aca gtc ctt cag cgc ctg atg ctc tca gat agc cac att tcc gcc       293
Met Thr Val Leu Gln Arg Leu Met Leu Ser Asp Ser His Ile Ser Ala
        75                  80                  85

atc gac ccc ggc acc ttc aat gac ctg gta aaa ctg aaa acc ctc agg       341
Ile Asp Pro Gly Thr Phe Asn Asp Leu Val Lys Leu Lys Thr Leu Arg
    90                  95                  100

ttg acg cgc aac aaa atc tct cgt ctt cca cgt gcg atc ctg gat aag       389
Leu Thr Arg Asn Lys Ile Ser Arg Leu Pro Arg Ala Ile Leu Asp Lys
105                 110                 115                 120

atg gta ctc ttg gaa cag ctg ttc ttg gac cac aat gca cta agg gac       437
Met Val Leu Leu Glu Gln Leu Phe Leu Asp His Asn Ala Leu Arg Asp
                125                 130                 135

ctt gat caa aac ctg ttt cag caa ctg cgt aac ctt cag gag ctc ggt       485
Leu Asp Gln Asn Leu Phe Gln Gln Leu Arg Asn Leu Gln Glu Leu Gly
```

```
            140                 145                 150

ttg aac cag aat cag ctc tct ttt ctt cct gct aac ctt ttc tcg agc          533
Leu Asn Gln Asn Gln Leu Ser Phe Leu Pro Ala Asn Leu Phe Ser Ser
        155                 160                 165

ctg aga gaa ctg aag ttg ttg gat tta tcg cga aac aac ctg acc cac          581
Leu Arg Glu Leu Lys Leu Leu Asp Leu Ser Arg Asn Asn Leu Thr His
    170                 175                 180

ctg ccc aag gga ctg ctt ggg gct caa gtt aag ctt gag aaa ctg ctg          629
Leu Pro Lys Gly Leu Leu Gly Ala Gln Val Lys Leu Glu Lys Leu Leu
185                 190                 195                 200

ctc tat tca aac cag ctc acg tct gtg gat tcg ggg ctg ctg agc aac          677
Leu Tyr Ser Asn Gln Leu Thr Ser Val Asp Ser Gly Leu Leu Ser Asn
                205                 210                 215

ctg ggc gcc ctg act gag ctg cgg ctg gag cgg aat cac ctc cgc tcc          725
Leu Gly Ala Leu Thr Glu Leu Arg Leu Glu Arg Asn His Leu Arg Ser
            220                 225                 230

gta gcc ccg ggt gcc ttc gac cgc ctc gga aac ctg agc tcc ttg act          773
Val Ala Pro Gly Ala Phe Asp Arg Leu Gly Asn Leu Ser Ser Leu Thr
        235                 240                 245

cta tcc gga aac ctc ctg gag tct ctg ccg ccc gcg ctc ttc ctt cac          821
Leu Ser Gly Asn Leu Leu Glu Ser Leu Pro Pro Ala Leu Phe Leu His
    250                 255                 260

gtg agc agc gtg tct cgg ctg act ctg ttc gag aac ccc ctg gag gag          869
Val Ser Ser Val Ser Arg Leu Thr Leu Phe Glu Asn Pro Leu Glu Glu
265                 270                 275                 280

ctc ccg gac gtg ttg ttc ggg gag atg gcc ggc ctg cgg gag ctg tgg          917
Leu Pro Asp Val Leu Phe Gly Glu Met Ala Gly Leu Arg Glu Leu Trp
                285                 290                 295

ctg aac ggc acc cac ctg agc acg ctg ccc gcc gct gcc ttc cgc aac          965
Leu Asn Gly Thr His Leu Ser Thr Leu Pro Ala Ala Ala Phe Arg Asn
            300                 305                 310

ctg agc ggc ttg cag acg ctg ggg ctg acg cgg aac ccg cgc ctg agc         1013
Leu Ser Gly Leu Gln Thr Leu Gly Leu Thr Arg Asn Pro Arg Leu Ser
        315                 320                 325

gcg ctc ccg cgc ggc gtg ttc cag ggc cta cgg gag ctg cgc gtg ctc         1061
Ala Leu Pro Arg Gly Val Phe Gln Gly Leu Arg Glu Leu Arg Val Leu
    330                 335                 340

gcg ctg cac acc aac gcc ctg gcg gag ctg cgg gac gac gcg ctg cgc         1109
Ala Leu His Thr Asn Ala Leu Ala Glu Leu Arg Asp Asp Ala Leu Arg
345                 350                 355                 360

ggc ctc ggg cac ctg cgc cag gtg tcg ctg cgc cac aac cgg ctg cgg         1157
Gly Leu Gly His Leu Arg Gln Val Ser Leu Arg His Asn Arg Leu Arg
                365                 370                 375

gcc ctg ccc cgc acg ctc ttc cgc aac ctc agc agc ctc gag agc gtg         1205
Ala Leu Pro Arg Thr Leu Phe Arg Asn Leu Ser Ser Leu Glu Ser Val
            380                 385                 390

cag cta gag cac aac cag ctg gag acg ctg cca gga gac gtg ttc gcg         1253
```

```
Gln Leu Glu His Asn Gln Leu Glu Thr Leu Pro Gly Asp Val Phe Ala
        395                 400                 405

gct ctg ccc cag ctg acc cag gtc ctg ctg ggt cac aac ccc tgg ctc      1301
Ala Leu Pro Gln Leu Thr Gln Val Leu Leu Gly His Asn Pro Trp Leu
    410                 415                 420

tgc gac tgt ggc ctg tgg ccc ttc ctc cag tgg ctg cgg cat cac ccg      1349
Cys Asp Cys Gly Leu Trp Pro Phe Leu Gln Trp Leu Arg His His Pro
425                 430                 435                 440

gac atc ctg ggc cga gac gag ccc ccg cag tgc cgt ggc ccg gag cca      1397
Asp Ile Leu Gly Arg Asp Glu Pro Pro Gln Cys Arg Gly Pro Glu Pro
                445                 450                 455

cgc gcc agc ctg tcg ttc tgg gag ctg ctg cag ggt gac ccg tgg tgc      1445
Arg Ala Ser Leu Ser Phe Trp Glu Leu Leu Gln Gly Asp Pro Trp Cys
            460                 465                 470

ccg gat cct cgc agc ctg cct ctc gac cct cca acc gaa aat gct ctg      1493
Pro Asp Pro Arg Ser Leu Pro Leu Asp Pro Pro Thr Glu Asn Ala Leu
        475                 480                 485

gaa gcc ccg gtt ccg tcc tgg ctg cct aac agc tgg cag tcc cag acg      1541
Glu Ala Pro Val Pro Ser Trp Leu Pro Asn Ser Trp Gln Ser Gln Thr
    490                 495                 500

tgg gcc cag ctg gtg gcc agg ggt gaa agt ccc aat aac agg ctc tac      1589
Trp Ala Gln Leu Val Ala Arg Gly Glu Ser Pro Asn Asn Arg Leu Tyr
505                 510                 515                 520

tgg ggt ctt tat att ctg ctt cta gta gcc cag gcc atc ata gcc gcg      1637
Trp Gly Leu Tyr Ile Leu Leu Leu Val Ala Gln Ala Ile Ile Ala Ala
                525                 530                 535

ttc atc gtg ttt gcc atg att aaa atc ggc cag ctg ttt cga aca tta      1685
Phe Ile Val Phe Ala Met Ile Lys Ile Gly Gln Leu Phe Arg Thr Leu
            540                 545                 550

atc aga gag aag ctc ttg tta gag gca atg gga aaa tcg tgt aac taa      1733
Ile Arg Glu Lys Leu Leu Leu Glu Ala Met Gly Lys Ser Cys Asn
        555                 560                 565

tgaaactgac cagagcattg tggacggggc cccaaggaga atgcagtcag gatgctggcg    1793

tgccattaca ctatttccca ggccttttct cctctcccgt gctcttagtg tctcttcttc    1853

tcccctctct tcagaagtag cttttgtaaa tcgctactgc tttctagcct ggcctgggtt    1913

acctcctctg ctgttagttt caagggggct gagggtgggg gttcgacggg acttggctca    1973

tcaggtccaa ctgtgcagcg ctgggtgcct agtggagaga ggagcccttt cttggtttct    2033

gaatttgagg acacatcctg ccagtgggca agacctctcc gggacccagc aagggttgag    2093

taacatttgc tgaaggaaca ccggcttaaa acgaacccta ggtccaagag atgaaggctc    2153

ttcccaaaat aaaggtggag tgttcttgtc cctttacctg aaaggaaaaa aaaaaaaaaa    2213

aa                                                                   2215
```

<210>  5

<211> 567
<212> PRT
<213> Mus musculus

<400> 5

```
Met Leu Arg Ser Ala Leu Leu Ser Ala Val Leu Ala Leu Leu Arg Ala
1               5                   10                  15

Gln Pro Phe Pro Cys Pro Lys Thr Cys Lys Cys Val Val Arg Asp Ala
            20                  25                  30

Ala Gln Cys Ser Gly Gly Ser Val Ala His Ile Ala Glu Leu Gly Leu
        35                  40                  45

Pro Thr Asn Leu Thr His Ile Leu Leu Phe Arg Met Asp Gln Gly Ile
    50                  55                  60

Leu Arg Asn His Ser Phe Ser Gly Met Thr Val Leu Gln Arg Leu Met
65                  70                  75                  80

Leu Ser Asp Ser His Ile Ser Ala Ile Asp Pro Gly Thr Phe Asn Asp
                85                  90                  95

Leu Val Lys Leu Lys Thr Leu Arg Leu Thr Arg Asn Lys Ile Ser Arg
            100                 105                 110

Leu Pro Arg Ala Ile Leu Asp Lys Met Val Leu Leu Glu Gln Leu Phe
        115                 120                 125

Leu Asp His Asn Ala Leu Arg Asp Leu Asp Gln Asn Leu Phe Gln Gln
    130                 135                 140

Leu Arg Asn Leu Gln Glu Leu Gly Leu Asn Gln Asn Gln Leu Ser Phe
145                 150                 155                 160

Leu Pro Ala Asn Leu Phe Ser Ser Leu Arg Glu Leu Lys Leu Leu Asp
                165                 170                 175

Leu Ser Arg Asn Asn Leu Thr His Leu Pro Lys Gly Leu Leu Gly Ala
            180                 185                 190

Gln Val Lys Leu Glu Lys Leu Leu Leu Tyr Ser Asn Gln Leu Thr Ser
        195                 200                 205

Val Asp Ser Gly Leu Leu Ser Asn Leu Gly Ala Leu Thr Glu Leu Arg
    210                 215                 220

Leu Glu Arg Asn His Leu Arg Ser Val Ala Pro Gly Ala Phe Asp Arg
```

225 230 235 240

Leu Gly Asn Leu Ser Ser Leu Thr Leu Ser Gly Asn Leu Leu Glu Ser
245 250 255

Leu Pro Pro Ala Leu Phe Leu His Val Ser Ser Val Ser Arg Leu Thr
260 265 270

Leu Phe Glu Asn Pro Leu Glu Glu Leu Pro Asp Val Leu Phe Gly Glu
275 280 285

Met Ala Gly Leu Arg Glu Leu Trp Leu Asn Gly Thr His Leu Ser Thr
290 295 300

Leu Pro Ala Ala Ala Phe Arg Asn Leu Ser Gly Leu Gln Thr Leu Gly
305 310 315 320

Leu Thr Arg Asn Pro Arg Leu Ser Ala Leu Pro Arg Gly Val Phe Gln
325 330 335

Gly Leu Arg Glu Leu Arg Val Leu Ala Leu His Thr Asn Ala Leu Ala
340 345 350

Glu Leu Arg Asp Asp Ala Leu Arg Gly Leu Gly His Leu Arg Gln Val
355 360 365

Ser Leu Arg His Asn Arg Leu Arg Ala Leu Pro Arg Thr Leu Phe Arg
370 375 380

Asn Leu Ser Ser Leu Glu Ser Val Gln Leu Glu His Asn Gln Leu Glu
385 390 395 400

Thr Leu Pro Gly Asp Val Phe Ala Ala Leu Pro Gln Leu Thr Gln Val
405 410 415

Leu Leu Gly His Asn Pro Trp Leu Cys Asp Cys Gly Leu Trp Pro Phe
420 425 430

Leu Gln Trp Leu Arg His His Pro Asp Ile Leu Gly Arg Asp Glu Pro
435 440 445

Pro Gln Cys Arg Gly Pro Glu Pro Arg Ala Ser Leu Ser Phe Trp Glu
450 455 460

Leu Leu Gln Gly Asp Pro Trp Cys Pro Asp Pro Arg Ser Leu Pro Leu
465 470 475 480

```
Asp Pro Pro Thr Glu Asn Ala Leu Glu Ala Pro Val Pro Ser Trp Leu
                485                 490                 495

Pro Asn Ser Trp Gln Ser Gln Thr Trp Ala Gln Leu Val Ala Arg Gly
            500                 505                 510

Glu Ser Pro Asn Asn Arg Leu Tyr Trp Gly Leu Tyr Ile Leu Leu Leu
        515                 520                 525

Val Ala Gln Ala Ile Ile Ala Ala Phe Ile Val Phe Ala Met Ile Lys
    530                 535                 540

Ile Gly Gln Leu Phe Arg Thr Leu Ile Arg Glu Lys Leu Leu Leu Glu
545                 550                 555                 560

Ala Met Gly Lys Ser Cys Asn
                565
```

<210> 6
<211> 551
<212> PRT
<213> Mus musculus

<400> 6

```
Gln Pro Phe Pro Cys Pro Lys Thr Cys Lys Cys Val Val Arg Asp Ala
1               5                   10                  15

Ala Gln Cys Ser Gly Gly Ser Val Ala His Ile Ala Glu Leu Gly Leu
            20                  25                  30

Pro Thr Asn Leu Thr His Ile Leu Leu Phe Arg Met Asp Gln Gly Ile
        35                  40                  45

Leu Arg Asn His Ser Phe Ser Gly Met Thr Val Leu Gln Arg Leu Met
    50                  55                  60

Leu Ser Asp Ser His Ile Ser Ala Ile Asp Pro Gly Thr Phe Asn Asp
65                  70                  75                  80

Leu Val Lys Leu Lys Thr Leu Arg Leu Thr Arg Asn Lys Ile Ser Arg
                85                  90                  95

Leu Pro Arg Ala Ile Leu Asp Lys Met Val Leu Leu Glu Gln Leu Phe
            100                 105                 110

Leu Asp His Asn Ala Leu Arg Asp Leu Asp Gln Asn Leu Phe Gln Gln
        115                 120                 125
```

```
Leu Arg Asn Leu Gln Glu Leu Gly Leu Asn Gln Asn Gln Leu Ser Phe
    130                 135                 140

Leu Pro Ala Asn Leu Phe Ser Ser Leu Arg Glu Leu Lys Leu Leu Asp
145                 150                 155                 160

Leu Ser Arg Asn Asn Leu Thr His Leu Pro Lys Gly Leu Leu Gly Ala
                165                 170                 175

Gln Val Lys Leu Glu Lys Leu Leu Leu Tyr Ser Asn Gln Leu Thr Ser
            180                 185                 190

Val Asp Ser Gly Leu Leu Ser Asn Leu Gly Ala Leu Thr Glu Leu Arg
        195                 200                 205

Leu Glu Arg Asn His Leu Arg Ser Val Ala Pro Gly Ala Phe Asp Arg
    210                 215                 220

Leu Gly Asn Leu Ser Ser Leu Thr Leu Ser Gly Asn Leu Leu Glu Ser
225                 230                 235                 240

Leu Pro Pro Ala Leu Phe Leu His Val Ser Ser Val Ser Arg Leu Thr
                245                 250                 255

Leu Phe Glu Asn Pro Leu Glu Glu Leu Pro Asp Val Leu Phe Gly Glu
            260                 265                 270

Met Ala Gly Leu Arg Glu Leu Trp Leu Asn Gly Thr His Leu Ser Thr
        275                 280                 285

Leu Pro Ala Ala Ala Phe Arg Asn Leu Ser Gly Leu Gln Thr Leu Gly
    290                 295                 300

Leu Thr Arg Asn Pro Arg Leu Ser Ala Leu Pro Arg Gly Val Phe Gln
305                 310                 315                 320

Gly Leu Arg Glu Leu Arg Val Leu Ala Leu His Thr Asn Ala Leu Ala
                325                 330                 335

Glu Leu Arg Asp Asp Ala Leu Arg Gly Leu Gly His Leu Arg Gln Val
            340                 345                 350

Ser Leu Arg His Asn Arg Leu Arg Ala Leu Pro Arg Thr Leu Phe Arg
        355                 360                 365

Asn Leu Ser Ser Leu Glu Ser Val Gln Leu Glu His Asn Gln Leu Glu
    370                 375                 380
```

```
Thr Leu Pro Gly Asp Val Phe Ala Ala Leu Pro Gln Leu Thr Gln Val
385                 390                 395                 400

Leu Leu Gly His Asn Pro Trp Leu Cys Asp Cys Gly Leu Trp Pro Phe
                405                 410                 415

Leu Gln Trp Leu Arg His His Pro Asp Ile Leu Gly Arg Asp Glu Pro
            420                 425                 430

Pro Gln Cys Arg Gly Pro Glu Pro Arg Ala Ser Leu Ser Phe Trp Glu
        435                 440                 445

Leu Leu Gln Gly Asp Pro Trp Cys Pro Asp Pro Arg Ser Leu Pro Leu
    450                 455                 460

Asp Pro Pro Thr Glu Asn Ala Leu Glu Ala Pro Val Pro Ser Trp Leu
465                 470                 475                 480

Pro Asn Ser Trp Gln Ser Gln Thr Trp Ala Gln Leu Val Ala Arg Gly
                485                 490                 495

Glu Ser Pro Asn Asn Arg Leu Tyr Trp Gly Leu Tyr Ile Leu Leu Leu
            500                 505                 510

Val Ala Gln Ala Ile Ile Ala Ala Phe Ile Val Phe Ala Met Ile Lys
        515                 520                 525

Ile Gly Gln Leu Phe Arg Thr Leu Ile Arg Glu Lys Leu Leu Leu Glu
    530                 535                 540

Ala Met Gly Lys Ser Cys Asn
545                 550
```

## Description

BACKGROUND

Platelet activation and subsequent thrombus formation at sites of vascular injury is crucial for normal hemostasis, but it can also cause myocardial infarction and stroke (Coughlin SR. Nature. 2000; 407: 258-64). Platelet adhesion and activation is a multistep process involving multiple platelet receptor-ligand interactions. Upon vessel wall injury, circulating platelets are rapidly decelerated by transient interactions of the glycoprotein (GP) Ib-V-IX complex with von Willebrand factor (vWF) immobilized on the exposed subendothelial extracellular matrix (e.g. on collagen) (Shapiro MJ, et al. JBC. 2000; 275: 25216-21). This interaction retains platelets close to the vessel wall and facilitates the contact between GPVI and collagen (Nieswandt B, et al. Blood. 2003; 102: 449-61.). GPVI-collagen interactions induce an intracellular signaling cascade leading to platelet activation and the release of secondary platelet agonists, such as thromboxane $A_2$ ($TxA_2$) and adenosine diphosphate (ADP). These soluble agonists together with locally produced thrombin further contribute to platelet activation through G protein ($G_i$, $G_q$, $G_{12/13}$) coupled receptors (Offermanns S. Circulation research. 2006; 99: 1293-304). All these signaling pathways synergize to induce complex cellular responses, such as activation of integrins, release of granule contents and the provision of a pro-coagulant surface for the activation of the coagulation cascade (Nakanishi-Matsui M, et al. Nature. 2000; 404: 609-13; Cunningham MA, et al. J Exp Med 2000; 191: 455-62). The final thrombus is embedded in a fibrin network to withstand the shear forces generated by the flowing blood. The stabilization of a newly formed thrombus is essential to arrest bleeding at sites of vascular injury.

Their central role in platelet adhesion puts two receptor complexes in the focus of platelet research: i) the GPIb-V-IX complex which interacts with vWF immobilized on the injured vessel wall or on activated platelets and thereby recruits platelets from the blood stream to the reactive surface under conditions of elevated shear. ii) GPIIb/IIIa (integrin αIIba3), a receptor for fibrinogen and vWF that requires inside-out activation mediated by agonist receptors, contributes to firm shear-resistant platelet adhesion and is essential for aggregate formation. The GPIb-V-IX complex is composed of 4 related transmembrane GPs: GPIbα, GPIbβ, GPV and GPIX, which are associated in a stoichiometry of 2:4:2:1(Luo S.-Z. et al., Blood, 2007. 109(2): 603-9). Within this complex, GPIbα and GPIbβ are disulfide-linked and noncovalently associated with GPIX. GPV is noncovalently associated with GPIb-IX (Nieswandt B, et al. J Thromb Haemost. 2009; 7: 206-9). Approximately 30,000 copies of the GPIb-IX complex are found on the surface of human platelets (Varga-Szabo D, et al. J Thromb Haemost. 2009; 7: 1057-66). Loss of GPIb-V-IX function causes Bernard-Soulier syndrome (BSS), a severe bleeding disorder. BSS is characterized by abnormal, giant circulating platelets with defective adhesion to vWF and reduced thrombin responsiveness (Canobbio I, et al. Cellular signalling. 2004; 16: 1329-44). While lack or dysfunction of GPIb or GPIX are associated with BSS, no loss of function mutation in GP5 has been reported and the lack of GPV in mice does not lead to a BSS-phenotype (Ramakrishnan V, et al. PNAS. 1999; 96: 13336-41; Kahn ML, et al. Blood. 1999; 94: 4112-21). GPV is the only subunit which is not required for the correct expression of the complex (Dong J-f, et al. J Biol Chem. 1998; 273: 31449-54). GPV is highly glycosylated and contains a thrombin cleavage site leading to quantitative removal of GPV from the platelet surface and the generation of soluble GPV (sGPV) in the presence of thrombin (Ravanat C, et al. Blood. 1997; 89: 3253-62; Azorsa DO, et al. Thrombosis and Haemostasis. 1999; 81: 131-8). Of note, this thrombin cleavage site is conserved in the mouse, rat and human protein (Ravanat C, et al. Blood. 1997; 89: 3253-62). However, in contrast to protease-activated receptor (PAR) 4-deficient mice, which do not respond upon thrombin stimulation (Kahn ML, et al. Blood. 1999; 94: 4112-21; Kahn ML, et al. Nature. 1998; 394: 690-4.), *Gp5*$^{-/-}$ mice display grossly normal platelet functionality.

*In vitro, Gp5*$^{-/-}$ platelets are hardly distinguishable from wildtype platelets (Ramakrishnan V, et al. PNAS. 1999; 96: 13336-41; Kahn ML, et al. Blood. 1999; 94: 4112-21). Only after activation with threshold doses of thrombin, an increased responsiveness was observed (Ramakrishnan V, et al. PNAS. 1999; 96: 13336-41), which has been ascribed to the lack of GPV as an alternative substrate for thrombin competing with PARs. For one of the two *GpS*$^{-/-}$ mouse strains, reduced tail bleeding times, accelerated thrombus formation and increased embolization were reported (Ramakrishnan V, et al. PNAS. 1999; 96: 13336-41; Ni H, et al. Blood. 2001; 98: 368-73), whereas analysis of the second mouse line revealed unaltered tail bleeding times and impaired thrombus formation (Kahn ML, et al. Blood. 1999; 94: 4112-21; Moog S, et al. Blood. 2001; 98: 1038-46). The latter group ascribed the defective thrombus formation to the role of GPV in collagen signaling, thereby establishing collagen as ligand for GPV (Moog S, et al. Blood. 2001; 98: 1038-46). The latest report on *GpS*$^{-/-}$ mice used mice backcrossed to the C57Bl/6 background and confirmed the increased thrombin responsiveness as well as slightly reduced adhesion on collagen. Using laser-injury, the authors demonstrated that the effect of GPV-deficiency on thrombus formation depends on the severity of the injury and concluded that GPV is only of minor relevance for arterial thrombus formation (Nonne C, et al. J Thromb Haemost. 2008; 6: 210-2). So far, the role of GPV in thrombosis and hemostasis seems to be of minor relevance for maintaining hemostasis and platelet function. However, the exact function of GPV in thrombosis, hemostasis and thrombo-inflammatory brain infarction still remains poorly understood.

SUMMARY OF THE INVENTION

The inventors surprisingly found that mice treated with an antibody directed against the extracellular domain of GPV displayed an accelerated time to thrombus formation *in vivo.* In addition, antibody-mediated blockade of GPV could fully compensate for the lack of GPVI in *in vivo* thrombus formation and hemostasis.

The present invention therefore relates to the following embodiments [1] to [25]:

[1] An inhibitor of glycoprotein V (GPV) for use as a coagulant.

[2] An inhibitor of glycoprotein V (GPV) for use in the treatment or prevention of a hemorrhagic condition.

[3] The inhibitor for use according to embodiment [2], wherein said hemorrhagic condition is caused by a platelet disorder.

[4] The inhibitor for use according to embodiment [3], wherein said platelet disorder is characterized by a decreased number of platelets.

[5] The inhibitor for use according to embodiment [3] or [4], wherein the platelet disorder is thromobocytopenia, e.g. idiopathic thrombocytopenic purpura, thrombotic thrombocytopenic purpura, thrombocytopenia caused by chemotherapy, or immunothrombocytopenia.

[6] The inhibitor for use according to any one of embodiments [2], wherein said hemorrhagic condition is selected from the group consisting of inflammatory bleeding, hemorrhagic stroke, excessive bleeding due to sepsis, excessive bleeding due to thrombocytopenia, excessive bleeding due to disseminated intravascular coagulation (DIC), excessive bleeding due to chemotherapy, excessive bleeding due to hemolytic-uremic syndrome, and excessive bleeding due to HIV infection.

[7] The inhibitor for use according to any one of the preceding embodiments, wherein said GPV is human GPV.

[8] The inhibitor for use according to embodiment [7], wherein said human GPV comprises or consists of the amino acid sequence as shown in SEQ ID NO:3.

[9] The inhibitor for use according to any one of the preceding embodiments, wherein said inhibitor is a polypeptide.

[10] The inhibitor for use according to embodiment [9], wherein said inhibitor is (i) an antibody capable of binding to the extracellular domain of GPV, (ii) a fragment or derivative of an antibody, said fragment or derivative being capable of binding to the extracellular domain of GPV, (iii) an antibody capable of binding to an epitope within the extracellular domain of GPV, or (iv) a fragment or derivative of an antibody, said fragment or derivative being capable of binding to an epitope within the extracellular domain of GPV.

[11] The inhibitor for use according to embodiment [10], wherein said extracellular domain comprises or consists of amino acids 1 to 503 of SEQ ID NO:3.

[12] The inhibitor for use according to embodiment [10] or [11], wherein said antibody is a monoclonal antibody or a functional fragment or functional derivative thereof.

[13] The inhibitor for use according to any one of the preceding embodiments, wherein said inhibitor is a nucleic acid.

[14] The inhibitor for use according to embodiment [13], wherein said nucleic acid is capable of reducing expression of GPV.

[15] The inhibitor for use according to embodiment [14], wherein said nucleic acid is selected from the group consisting of antisense nucleic acid, siRNA and shRNA.

[16] The inhibitor for use according to embodiment [13], wherein said nucleic acid is capable of binding to the extracellular domain of GPV.

[17] The inhibitor for use according to embodiment [16], wherein said nucleic acid is an aptamer.

[18] The inhibitor for use according to any one of the preceding embodiments, wherein said inhibitor, upon administration to a subject, does not substantially affect the number of platelets in said subject.

[19] The inhibitor for use according to any one of embodiments [2] to [18], wherein said inhibitor is used as a coagulant.

[20] The inhibitor for use according to any one of embodiments [2] to [19], wherein said treatment or prevention comprises administering to a subject, preferably to a human, a pharmaceutically effective amount of said inhibitor.

[21] The inhibitor for use according to embodiment [20], wherein said treatment or prevention further comprises administering to said subject a coagulant other than said inhibitor.

[22] The inhibitor for use according to embodiment [21], wherein said coagulant other than said inhibitor is selected from the group consisting of an antifibrinolytic agent, a platelet concentrate, a coagulation factor concentrate and fresh frozen plasma.

[23] A pharmaceutical composition comprising an inhibitor as defined in any one of the preceding embodiments, and a pharmaceutically acceptable excipient.

[24] A pharmaceutical composition as defined in embodiment [23] for use in in the treatment or prevention of a hemorrhagic condition.

[25] A method of treating a hemorrhagic condition in a subject, preferably a human, comprising administering to the subject an effective amount of an inhibitor as defined in any one of embodiments [1] to [18], or of the pharmaceutical composition of embodiment [23].

BRIEF DESCRIPTION OF THE DRAWINGS

**Figure** 1: **The monoclonal anti-GPV antibody 89F12 binds to the extracellular domain of GPV.** (A) 89F12-FITC binds to WT, but not to GPV-deficient platelets as assessed by flow cytometry. (B) 89F12-HRP detects recombinant murine sGPV in an ELISA system.

**Figure 2: The monoclonal anti-GPV antibody 89F12 binds to the extracellular domain of GPV without affecting platelet count and thrombin-mediated cleavage of GPV.** (A, B) 100 μg 89F12 was injected intravenously and afterwards, platelet count as well as GPV surface expression were analyzed. (C) The antibody 89F12 does not affect the thrombin-mediated cleavage of GPV as assessed by flow cytometry. (D) 89F12 does not alter $\alpha$IIb$\beta$3 integrin-mediated platelet activation and P-selectin exposure in response to thrombin.

**Figure 3: The antibody 89F12 propagates hemostasis in wildtype mice and restores** it **in GPVI/CLEC-2 double-deficient animals.** Displayed are tail bleeding times of the indicated mouse lines. Each symbol represents one animal. In cases where mice were unable to arrest bleeding Fisher's exact test was used to calculate P-values.

**Figure 4: The antibody 89F12 restores hemostasis in GPVI/ITGA2 double-deficient mice.** Displayed are tail bleeding times of the indicated mouse lines. Each symbol represents one animal. In cases where mice were unable to arrest bleeding Fisher's exact test was used to calculate P-values.

**Figure 5: The monoclonal anti-GPV antibody 89F12 compensates for the lack of GPVI in an *in vivo* thrombus formation model.** Mesenteric arterioles were injured with 20% $FeCl_3$ and adhesion and thrombus formation of fluorescently-labeled platelets were monitored by intravital microscopy. Each dot represents one vessel, horizontal lines indicate the median. * P<0.05; ** P<0.01; *** P<0.001.

**Figure 6: The antibody 89F12 can compensate for the lack of RhoA in hemostasis.** Displayed are tail bleeding times of the indicated mouse lines. Each symbol represents one animal, horizontal lines indicate the median (not depicted if the median would have been above 1200 s). In cases where mice were unable to arrest bleeding Fisher's exact test was used to calculate P-values. * *P*<0.05; ** *P*<0.01.

DETAILED DESCRIPTION

The present invention relates to an inhibitor of glycoprotein V (GPV) for use as a coagulant, and/or to an inhibitor of GPV for use in the treatment or prevention of a hemorrhagic condition.

*Glycoprotein V*

The term "Glycoprotein V" or "GPV", as used herein, denotes a membrane protein having a sequence identity of at least 50% to the amino acid sequence as shown in SEQ ID NO:3. Preferably, the GPV has an amino acid identity of at least 60%, or at least 70%, or at least 80%, or at least 90%, or at least 95% to the amino acid sequence as shown in SEQ ID NO:3. The GPV has a functional transmembrane domain.

In accordance with the present invention, a sequence being evaluated (the "Compared Sequence") has a certain "percent identity with", or is certain "percent identical to" a claimed or described sequence (the "Reference Sequence") after alignment of the two sequences. The "Percent Identity" is determined according to the following formula:

$$\text{Percent Identity}=100[1-(C/R)]$$

In this formula, C is the number of differences between the Reference Sequence and the Compared Sequence over the length of alignment between the two sequences wherein (i) each base in the Reference Sequence that does not have a corresponding aligned base in the Compared Sequence, and (ii) each gap in the Reference Sequence, and (iii) each aligned base in the Reference Sequence that is different from an aligned base in the Compared Sequence constitutes a difference. R is the number of bases of the Reference Sequence over the length of the alignment with the Compared Sequence with any gap created in the Reference Sequence also being counted as a base.

If an alignment exists between the Compared Sequence and the Reference Sequence for which the Percent Identity (calculated as above) is about equal to, or greater than, a specified minimum, the Compared Sequence has that specified minimum Percent Identity even if alignments may exist elsewhere in the sequence that show a lower Percent Identity than that specified.

In a preferred embodiment, the length of aligned sequence for comparison purposes is at least 30%, preferably at least 40%, more preferably at least 50%, even more preferably at least 60%, and even more preferably at least 70%, 80%, or 90% of the length of the Reference Sequence.

The comparison of sequences and determination of percent identity (and percent similarity) between two amino acid sequences can be accomplished using any suitable program, e.g. the program "BLAST 2 SEQUENCES (blastp)" (Tatusova et al. FEMS Microbiol. Lett. 1999; 174: 247-250) with the following parameters: Matrix BLOSUM62; Open gap 11 and extension gap 1 penalties; gap x_dropoff50; expect 10.0 word size 3; Filter: none. According to the present invention, the sequence comparison covers at least 40 amino acids, preferably at least 80 amino acids, more preferably at least 100 amino acids, and most preferably at least 120 amino acids.

Typically, the GPV is a naturally occurring GPV. Preferably, the GPV is of mammalian origin. Most preferably, the GPV is a human GPV. According to this embodiment, the GPV preferably comprises or consists of the amino acid sequence as shown in SEQ ID NO:3.

*Inhibitors of GPV*

An inhibitor of GPV (hereinafter referred to as "GPV inhibitor") is a compound which (i) has pro-coagulatory activity and (ii) is capable of binding to the extracellular domain of GPV, or of attenuating expression of GPV mRNA. Preferably, the GPV inhibitor is a compound which (i) has pro-coagulatory activity and (ii) is capable of binding to the extracellular domain of GPV. In accordance with this invention, pro-coagulatory activity is determined in a "Bleeding Time Assay" as described in the examples, with the proviso that the mouse used in the Bleeding Time Assay is a transgenic mouse lacking endogenous GPV and expressing human GPV. Binding to the extracellular domain of GPV can be determined in an ELISA as described in the examples. In one embodiment, binding to the extracellular domain of GPV is determined in an ELISA using recombinant soluble GPV, as depicted in Figure 1B, with the proviso that recombinant soluble *human* GPV is used. Most preferably, binding to the extracellular domain of GPV is determined in an ELISA using recombinant soluble GPV, as depicted in Figure 1B, with the proviso that recombinant soluble human GPV substantially consisting of amino acids 1-503 of SEQ ID NO:3 is used.

The GPV inhibitor may affect, e.g. inhibit, the thrombin-mediated cleavage of GPV in a subject upon administration of the GPV inhibitor to the subject. In another embodiment, the GPV inhibitor does not affect the thrombin-mediated cleavage of GPV in a subject upon administration of the GPV inhibitor to the subject.

The type or class of the GPV inhibitor is not particularly limited. Preferably, however, the compound is a peptide or polypeptide, more preferably the compound is an antibody or a fragment thereof. In yet another embodiment, the GPV inhibitor is a nucleic acid.

*Antibodies*

In a preferred embodiment, the GPV inhibitor is an antibody. The term "antibody", as used herein, refers to an immunoglobulin molecule that binds to or is immunologically reactive with a particular antigen, and includes polyclonal, monoclonal, genetically engineered and otherwise modified forms of antibodies including, but not limited to, chimeric antibodies, humanized antibodies, human antibodies, heteroconjugate antibodies (e.g. bispecific antibodies, diabodies, triabodies, and tetrabodies), single-domain antibodies (nanobodies) and antigen binding fragments of antibodies, including e.g. Fab', F(ab')2, Fab, Fv, rIgG, and scFv fragments. Moreover, unless otherwise indicated, the term "monoclonal antibody" (mAb) is meant to include both intact molecules, as well as, antibody fragments (such as, for example, Fab and F(ab')2 fragments) which are capable of binding to a protein. Fab and F(ab')2 fragments lack the Fc fragment of intact antibody, clear more rapidly from the circulation of the animal, and may have less non-specific tissue binding than an intact antibody (Wahl et al. J. Nucl. Med. 1983; 24: 316).

Typically, the antibody is capable of binding to the extracellular domain of GPV, preferably of human GPV. Whether a molecule or an antibody is capable of binding to the extracellular domain of GPV can be determined by a binding assay described in Example/Figure 1A or 1 B (see below).

The antibody preferably binds to an epitope within amino acids 1-503 of SEQ ID NO:3. For example, the present invention includes, but is not limited to, the following embodiments:

Table 1.

| Embodiment No. | The antibody binds to an epitope within the following amino acids of SEQ ID NO:3 |
|---|---|
| 1 | 1-30 |
| 2 | 16-45 |
| 3 | 31-60 |
| 4 | 46-75 |
| 5 | 61-90 |
| 6 | 76-105 |
| 7 | 91-120 |
| 8 | 106-135 |
| 9 | 121-150 |
| 10 | 136-165 |
| 11 | 151-180 |
| 12 | 166-195 |
| 13 | 181-210 |
| 14 | 196-225 |
| 15 | 211-240 |
| 16 | 226-255 |
| 17 | 241-270 |
| 18 | 256-285 |
| 19 | 271-300 |
| 20 | 286-315 |
| 21 | 301-330 |
| 22 | 316-345 |
| 23 | 331-360 |

(continued)

| Embodiment No. | The antibody binds to an epitope within the following amino acids of SEQ ID NO:3 |
|---|---|
| 24 | 346-375 |
| 25 | 361-390 |
| 26 | 376-405 |
| 27 | 391-420 |
| 28 | 406-435 |
| 29 | 421-450 |
| 30 | 436-465 |
| 31 | 451-480 |
| 32 | 466-495 |
| 33 | 481-503 |

The dissociation constant $K_D$ for the complex formed by the extracellular domain of GPV and antibody is preferably less than 100 μM, more preferably less than 10 μM, most preferably less than 5 μM. Typically the $K_D$ ranges from about 1 pM to about 10 μM, or from about 10 pM to about 1 μM, or from about 100 pM to about 100 nM. Preferably, the antibody-GPV complex has a $K_D$ in the range from 5 pM to 1 nM, most preferably from 10 pM to 500 pM.

Preferably, the antibody is a monoclonal antibody. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technologies, or a combination thereof (Harlow and Lane, "Antibodies, A Laboratory Manual" CSH Press 1988, Cold Spring Harbor N.Y.).

In other embodiments, including *in vivo* use of the anti-GPV antibodies in humans, chimeric, primatized, humanized, or human antibodies can be used. In a preferred embodiment, the antibody is a human antibody or a humanized antibody, more preferably a monoclonal human antibody or a monoclonal humanized antibody.

The term "chimeric" antibody as used herein refers to an antibody having variable sequences derived from non-human immunoglobulins, such as rat or mouse antibodies, and human immunoglobulins constant regions, typically chosen from a human immunoglobulin template. Methods for producing chimeric antibodies are known in the art. See, e.g. Morrison. Science. 1985; 229(4719): 1202-7; Oi et al. BioTechniques. 1986; 4: 214-221; Gillies et al. J. Immunol. Methods 1985; 125: 191-202; US 5,807,715; US 4,816,567; and US 4,816,397, which are incorporated herein by reference in their entireties.

"Humanized" forms of non-human (e.g. murine) antibodies are chimeric immunoglobulins, immunoglobulin chains or fragments thereof (such as Fv, Fab, Fab', F(ab')2 or other target-binding subsequences of antibodies), which contain minimal sequences derived from a non-human immunoglobulin. In general, the humanized antibody will comprise substantially all of at least one and typically two variable domains, in which all or substantially all of the complementarity determining regions (CDRs) correspond to those of a non-human immunoglobulin and all or substantially all of the framework (FR) regions are those of a human immunoglobulin consensus sequence. The humanized antibody can also comprise at least a portion of an immunoglobulin constant region (Fc), typically that of a human immunoglobulin template chosen. Humanization is a technique for making a chimeric antibody in which one or more amino acids or portions of the human variable domain have been substituted by the corresponding sequence from a non-human species. Humanized antibodies are antibody molecules generated in a non-human species that bind the desired antigen having one or more CDRs from the non-human species and FRs from a human immunoglobulin molecule. Often, framework residues in the human framework regions will be substituted with the corresponding residue from the CDR donor antibody to alter, preferably improve, antigen binding. These framework substitutions are identified by methods well known in the art, e.g. by modeling of the interactions of the CDR and framework residues to identify framework residues important for antigen binding and sequence comparison to identify unusual framework residues at particular positions. See, e.g. Riechmann et al. Nature 1988. 332: 323-7 and Queen et al. US 5,530,101; US 5,585,089; US 5,693,761; US 5,693,762; and US 6,180,370 (each of which is incorporated by reference in its entirety). Antibodies can be humanized using a variety of techniques known in the art including, for example, CDR-grafting (EP239400; WO 91/09967; US 5,225,539; US 5,530,101 and US 5,585,089), veneering or resurfacing (EP0592106; EP0519596; Padlan. Mol Immunol. 1991; 28: 489-498; Studnicka et al. Prot Eng. 1994; 7: 805-814; Roguska et al. PNAS 1994; 91: 969-973, and chain shuffling (US 5,565,332)), all of which are hereby incorporated by reference in their entireties.

In some embodiments, humanized antibodies are prepared as described in Queen et al., US 5,530,101; US 5,585,089; US 5,693,761; US 5,693,762; and US 6,180,370 (each of which is incorporated by reference in its entirety).

In some embodiments, the anti-GPV antibodies are human antibodies. Completely "human" anti-GPV antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods described above using antibody libraries derived from human immunoglobulin sequences. See US 4,444,887 and US 4,716,111; and WO 98/46645; WO 98/50433; WO 98/24893; WO 98/16654; WO 96/34096; WO 96/33735; and WO 91/10741, each of which is incorporated herein by reference in its entirety. Human antibodies can also be produced using transgenic mice which are incapable of expressing functional endogenous immunoglobulins, but which can express human immunoglobulin genes. See, e.g. WO 98/24893; WO 92/01047; WO 96/34096; WO 96/33735; US 5,413,923; US 5,625,126; US 5,633,425; US 5,569,825; US 5,661,016; US 5,545,806; US 5,814,318; US 5,885,793; US 5,916,771; and US 5,939,598, which are incorporated by reference herein in their entireties. Completely human antibodies that recognize a selected epitope can be generated using a technique referred to as "guided selection." In this approach a selected non-human monoclonal antibody, e.g. a mouse antibody, is used to guide the selection of a completely human antibody recognizing the same epitope (Jespers et al. Biotechnology. 1988; 12: 899-903).

In some embodiments, the anti-GPV antibodies are primatized antibodies. The term "primatized antibody" refers to an antibody comprising monkey variable regions and human constant regions. Methods for producing primatized antibodies are known in the art. See e.g. US 5,658,570; US 5,681,722; and US 5,693,780, which are incorporated herein by reference in their entireties.

In some embodiments, the anti-GPV antibodies are derivatized antibodies. For example, but not by way of limitation, the derivatized antibodies that have been modified, e.g. by glycosylation, acetylation, pegylation, phosphorylation, amidation, derivatization by known protecting/blocking groups, proteolytic cleavage or linkage to a cellular ligand or other proteins (see *below* for a discussion of antibody conjugates). Any of numerous chemical modifications may be carried out by known techniques, including, but not limited to, specific chemical cleavage, acetylation, formylation or metabolic synthesis of tunicamycin, etc. Additionally, the derivative may contain one or more non-classical amino acids.

In some embodiments, the anti-GPV antibodies or fragments thereof can be antibodies or antibody fragments, whose sequence has been modified to reduce at least one constant region-mediated biological effector function relative to the corresponding wild type sequence. To modify an anti-GPV antibody, such that it exhibits reduced binding to the Fc receptor, the immunoglobulin constant region segment of the antibody can be mutated at particular regions necessary for Fc receptor (FcR) interactions (see, e.g. Canfield and Morrison. J Exp Med. 1991; 173: 1483- 1491; and Lund et al. J Immunol. 1991; 147: 2657-2662). Reduction in FcR binding ability of the antibody can also reduce other effector functions which rely on FcR interactions, such as opsonization, phagocytosis and antigen-dependent cellular cytotoxicity.

In yet another aspect, the anti-GPV antibodies or fragments thereof can be antibodies or antibody fragments that have been modified to increase or reduce their binding affinities to the fetal Fc receptor, FcRn. To alter the binding affinity to FcRn, the immunoglobulin constant region segment of the antibody can be mutated at particular regions necessary for FcRn interactions (see, e.g. WO 2005/123780). Increasing the binding affinity to FcRn should increase the antibody's serum half-life, and reducing the binding affinity to FcRn should conversely reduce the antibody's serum half-life. Specific combinations of suitable amino acid substitutions are identified in Table 1 of WO 2005/123780, which table is incorporated by reference herein in its entirety. See also, Hinton et al., US 7,217,797, US 7,361,740, US 7,365,168, and US 7,217,798, which are incorporated herein by reference in their entireties. In yet other aspects, an anti-GPV antibody has one or more amino acids inserted into one or more of its hypervariable regions, for example as described in US 2007/0280931.

*Antibody Conjugates*

In some embodiments, the anti-GPV antibodies are antibody conjugates that are modified, e.g. by the covalent attachment of any type of molecule to the antibody, such that covalent attachment does not interfere with binding to GPV. Techniques for conjugating effector moieties to antibodies are well known in the art (See, e.g. Hellstrom et al., Controlled Drag Delivery, 2nd Ed., 623-53 (Robinson et al., eds., 1987); Thorpe et al. Immunol Rev. 1982; 62: 119-58 and Dubowchik et al. Pharmacology and Therapeutics 1999; 83: 67-123).

In one example, the antibody or fragment thereof is fused via a covalent bond (e.g. a peptide bond), at optionally the N-terminus or the C-terminus, to an amino acid sequence of another protein (or portion thereof; preferably at least a 10, 20 or 50 amino acid portion of the protein). Preferably, the antibody or fragment thereof is linked to the other protein at the N-terminus of the constant domain of the antibody. Recombinant DNA procedures can be used to create such fusions, for example as described in WO 86/01533 and EP 0392745. In another example, the effector molecule can increase half-life *in vivo.* Examples of suitable effector molecules of this type include polymers, albumin, albumin binding proteins or albumin binding compounds, such as those described in WO 2005/117984.

In some embodiments, anti-GPV antibodies can be attached to poly(ethyleneglycol) (PEG) moieties. For example, if the antibody is an antibody fragment, the PEG moieties can be attached through any available amino acid side-chain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. See, for example US 5,219,996. Multiple sites can be used to attach two or more PEG molecules. Preferably, PEG moieties are covalently linked through a thiol group of at least one cysteine residue located in the antibody fragment. Where a thiol group is used as the point of attachment, appropriately activated effector moieties, for example thiol selective derivatives, such as maleimides and cysteine derivatives, can be used.

In another example, an anti-GPV antibody conjugate is a modified Fab' fragment which is PEGylated, i.e., has PEG (poly(ethyleneglycol)) covalently attached thereto, e.g. according to the method disclosed in EP 0948544. See also Poly(ethyleneglycol) Chemistry, Biotechnical and Biomedical Applications, (J. Milton Harris (ed.), Plenum Press, New York, 1992); Poly(ethyleneglycol) Chemistry and Biological Applications, (J. Milton Harris and S. Zalipsky, eds., American Chemical Society, Washington D. C, 1997); and Bioconjugation Protein Coupling Techniques for the Biomedical Sciences, (M. Aslam and A. Dent, eds., Grove Publishers, New York, 1998); and Chapman. Advanced Drug Delivery Reviews 2002; 54: 531-545.

*Nucleic Acids*

In other embodiments of the invention, the GPV inhibitor is a nucleic acid. In a particular embodiment, the nucleic acid is capable of attenuating expression of GPV mRNA. Expression of GPV can be inhibited or reduced or abolished by RNA interference, e.g. by using siRNA or shRNA. Another possibility is the use of antisense nucleic acid, e.g. antisense RNA.

The phrase "attenuating expression of an mRNA," as used herein, means administering or expressing an amount of interfering RNA (e.g. an siRNA) to reduce translation of the target mRNA into protein, either through mRNA cleavage or through direct inhibition of translation.

The reduction in expression of the target mRNA or the corresponding protein is commonly referred to as "knock-down" and is reported relative to levels present following administration or expression of a non-targeting control RNA (e.g. a non-targeting control siRNA). Knock-down of expression of an amount including and between 50% and 100% is contemplated by embodiments herein. However, it is not necessary that such knock-down levels are achieved for purposes of the present invention. Knock-down is commonly assessed by measuring the mRNA levels using quantitative polymerase chain reaction (qPCR) amplification or by measuring protein levels by western blot or enzyme-linked immunosorbent assay (ELISA). Analyzing the protein level provides an assessment of both mRNA cleavage as well as translation inhibition. Further techniques for measuring knock-down include RNA solution hybridization, nuclease protection, northern hybridization, gene expression monitoring with a microarray, antibody binding, radioimmunoassay, and fluorescence activated cell analysis.

Inhibition of GPV mRNA expression may also be determined *in vitro* by evaluating GPV mRNA levels or GPV protein levels in, for example, human cells following transfection of GPV-interfering RNA.

In one embodiment of the invention, interfering RNA (e.g. siRNA) has a sense strand and an antisense strand, and the sense and antisense strands comprise a region of at least near-perfect contiguous complementarity of at least 19 nucleotides. In a further embodiment of the invention, interfering RNA (e.g. siRNA) has a sense strand and an antisense strand, and the antisense strand comprises a region of at least near-perfect contiguous complementarity of at least 19 nucleotides to a target sequence of GPV mRNA, and the sense strand comprises a region of at least near-perfect contiguous identity of at least 19 nucleotides with a target sequence of GPV mRNA, respectively. In a further embodiment of the invention, the interfering RNA comprises a region of at least 13, 14, 15, 16, 17, or 18 contiguous nucleotides having percentages of sequence complementarity to or, having percentages of sequence identity with, the penultimate 13, 14, 15, 16, 17, or 18 nucleotides, respectively, of the 3' end of the corresponding target sequence within an mRNA.

The length of each strand of the interfering RNA comprises 19 to 49 nucleotides, and may comprise a length of 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49 nucleotides. The antisense strand of an siRNA is the active guiding agent of the siRNA in that the antisense strand is incorporated into RISC, thus allowing RISC to identify target mRNAs with at least partial complementary to the antisense siRNA strand for cleavage or translational repression. In embodiments of the present invention, interfering RNA target sequences (e.g., siRNA target sequences) within the GPV mRNA sequence are selected using available design tools. Interfering RNAs corresponding to a GPV target sequence are then tested by transfection of cells expressing the target mRNA followed by assessment of knockdown as described above. Techniques for selecting target sequences for siRNAs are provided in "siRNA Design: Methods and Protocols", edited by Debra J. Taxman, 2012 (ISBN-13: 9781627031189).

In a second particular embodiment, the nucleic acid is capable of binding to the extracellular domain of human GPV. In accordance with this embodiment, the nucleic acid is preferably an aptamer. Aptamers can be designed as described in "De novo Molecular Design", 2013, edited by Gisbert Schneider, Chapter 21 (ISBN-13: 9783527677030); or in "The Aptamer Handbook: Functional Oligonucleotides and Their Applications", 2006, edited by Sven Klussmann (ISBN-13: 9783527607914).

*Hemorrhagic conditions*

The inhibitor described herein is preferably used in the treatment or prevention of a hemorrhagic condition. Hemorrhagic conditions are characterized by excessive bleeding. The excessive bleeding can have various causes.

In one embodiment, the hemorrhagic condition is caused by a platelet disorder.

The platelet disorder may be characterized by a decreased number of platelets, e.g. in the case of thrombocytopenia. Specific thrombocytopenias include, but are not limited to, idiopathic thrombocytopenic purpura, thrombotic thrombocytopenic purpura, drug-induced thrombocytopenia due to immune-mediated platelet destruction (e.g. by heparin, trimethoprim/sulfamethoxazole), drug-induced thrombocytopenia due to dose-dependent bone marrow suppression (e.g. by chemotherapeutic agents), thrombocytopenia accompanying systemic infection, thrombocytopenia caused by chemotherapy, gestational thrombocytopenia, and immune thrombocytopenia (ITP, formerly called immune thrombocytopenic purpura).

The platelet disorder may be characterized by a dysfunction of the platelets, e.g. in the case of defective platelet signaling due to lack of platelet receptors or signaling molecules.

In a preferred embodiment, the hemorrhagic condition is selected from the group consisting of inflammatory bleeding, hemorrhagic stroke, excessive bleeding due to sepsis, excessive bleeding due to thrombocytopenia, excessive bleeding due to disseminated intravascular coagulation (DIC), excessive bleeding due to chemotherapy, excessive bleeding due to hemolytic-uremic syndrome, and excessive bleeding due to HIV infection.

*Pharmaceutical Compositions and Treatment*

Treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom a GPV inhibitor, e.g. an anti-GPV antibody, is administered can be a mammal, such as a non-primate (e.g. cow, pig, horse, cat, dog, rat, etc.) or a primate (e.g. monkey or human). In certain aspects, the human is a pediatric patient. In other aspects, the human is an adult patient.

Compositions comprising a GPV inhibitor and, optionally one or more additional therapeutic agents, such as the second therapeutic agents described below, are described herein. The compositions typically are supplied as part of a sterile, pharmaceutical composition that includes a pharmaceutically acceptable carrier. This composition can be in any suitable form (depending upon the desired method of administering it to a patient).

The GPV inhibitors, e.g. the anti-GPV antibodies, can be administered to a patient by a variety of routes such as orally, transdermally, subcutaneously, intranasally, intravenously, intramuscularly, intrathecally, topically or locally. The most suitable route for administration in any given case will depend on the particular antibody, the subject, and the nature and severity of the disease and the physical condition of the subject. Typically, a GPV inhibitor, e.g. an anti-GPV antibody, will be administered intravenously.

Another aspect of the invention is a pharmaceutical composition comprising the antibody or antigen-binding fragment thereof of the invention. The antibody or antigen-binding fragment thereof can be formulated according to known methods for preparing a pharmaceutical composition. For example, it can be mixed with one or more pharmaceutically acceptable carriers, diluents or excipients. For example, sterile water or physiological saline may be used. Other substances, such as pH buffering solutions, viscosity reducing agents, or stabilizers may also be included.

A wide variety of pharmaceutically acceptable excipients and carriers are known in the art. Such pharmaceutical carriers and excipients as well as suitable pharmaceutical formulations have been amply described in a variety of publications (see for example "Pharmaceutical Formulation Development of Peptides and Proteins", Frokjaer et al., Taylor & Francis (2000) or "Handbook of Pharmaceutical Excipients", 3rd edition, Kibbe et al., Pharmaceutical Press (2000) A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., eds 7th ed., Lippincott, Williams, & Wilkins; and Handbook of Pharmaceutical Excipients (2000) A. H. Kibbe et al., eds., 3rd ed. Amer. Pharmaceutical Assoc). In particular, the pharmaceutical composition comprising the antibody of the invention may be formulated in lyophilized or stable soluble form. The polypeptide may be lyophilized by a variety of procedures known in the art. Lyophilized formulations are reconstituted prior to use by the addition of one or more pharmaceutically acceptable diluents such as sterile water for injection or sterile physiological saline solution.

The pharmaceutical composition of the invention can be administered in dosages and by techniques well known in the art. The amount and timing of the administration will be determined by the treating physician or veterinarian to achieve the desired purposes. The route of administration can be via any route that delivers a safe and therapeutically effective dose to the blood of the subject to be treated. Possible routes of administration include systemic, topical, enteral and parenteral routes, such as intravenous, intraarterial, subcutaneous, intradermal, intraperitoneal, oral, transmucosal, epidural, or intrathecal. Preferred routes are intravenous or subcutaneous.

The effective dosage and route of administration are determined by factors, such as age and weight of the subject, and by the nature and therapeutic range of the antibody or antigen-binding fragment thereof. The determination of the dosage is determined by known methods, no undue experimentation is required.

A therapeutically effective dose is a dose of the antibody or antigen binding fragment thereof of the invention that brings about a positive therapeutic effect in the patient or subject requiring the treatment. A therapeutically effective dose is in the range of about 0.01 to 50 mg/kg, from about 0.01 to 30 mg/kg, from about 0.1 to 30 mg/kg, from about 0.1 to 10 mg/kg, from about 0.1 to 5 mg/kg, from about 1 to 5 mg/kg, from about 0.1 to 2 mg/kg or from about 0.1 to 1 mg/kg. The treatment may comprise giving a single (e.g. bolus) dose or multiple doses. Alternatively continuous administration is possible. If multiple doses are required, they may be administered daily, every other day, weekly, biweekly, monthly, or bimonthly or as required. A depository may also be used that slowly and continuously releases the antibody or antigen-binding fragment thereof. A therapeutically effective dose may be a dose that inhibits GPV in the subject by at least 50%, preferably by at least 60%, 70%, 80%, 90%, more preferably by at least 95%, 99% or even 100%.

The antibody can be formulated as an aqueous solution.

Pharmaceutical compositions can be conveniently presented in unit dose forms containing a predetermined amount of a GPV inhibitor, e.g. an anti-GPV antibody, per dose. Such a unit can contain 0.5 mg to 5 g, for example, but without limitation, 1 mg, 10 mg, 20 mg, 30 mg, 40 mg, 50 mg, 100 mg, 200 mg, 300 mg, 400 mg, 500 mg, 750 mg, 1000 mg, or any range between any two of the foregoing values, for example 10 mg to 1000 mg, 20 mg to 50 mg, or 30 mg to 300 mg. Pharmaceutically acceptable carriers can take a wide variety of forms depending, e.g. on the condition to be treated or route of administration.

Determination of the effective dosage, total number of doses and length of treatment with a GPV inhibitor, e.g. an anti-GPV antibody, is well within the capabilities of those skilled in the art and can be determined using a standard dose escalation study.

Therapeutic formulations of the GPV inhibitors, e.g. the anti-GPV antibodies, suitable in the methods described herein can be prepared for storage as lyophilized formulations or aqueous solutions by mixing the inhibitor, e.g. the antibody, having the desired degree of purity with optional pharmaceutically-acceptable carriers, excipients or stabilizers typically employed in the art (all of which are referred to herein as "carriers"), i.e. buffering agents, stabilizing agents, preservatives, isotonifiers, non-ionic detergents, antioxidants and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentrations ranging from about 2 mM to about 50 mM. Suitable buffering agents include both organic and inorganic acids and salts thereof, such as citrate buffers (e.g. monosodium citrate-disodium citrate mixture, citric acid-trisodium citrate mixture, citric acid-monosodium citrate mixture, etc.), succinate buffers (e.g. succinic acid-monosodium succinate mixture, succinic acid-sodium hydroxide mixture, succinic acid-disodium succinate mixture, etc.), tartrate buffers (e.g. tartaric acid-sodium tartrate mixture, tartaric acid-potassium tartrate mixture, tartaric acid-sodium hydroxide mixture, etc.), fumarate buffers (e.g. fumaric acid-monosodium fumarate mixture, fumaric acid-disodium fumarate mixture, monosodium fumarate-disodium fumarate mixture, etc.), gluconate buffers (e.g. gluconic acid-sodium glyconate mixture, gluconic acid-sodium hydroxide mixture, gluconic acid-potassium glyuconate mixture, etc.), oxalate buffer (e.g. oxalic acid-sodium oxalate mixture, oxalic acid-sodium hydroxide mixture, oxalic acid-potassium oxalate mixture, etc), lactate buffers (e.g. lactic acid-sodium lactate mixture, lactic acid-sodium hydroxide mixture, lactic acid-potassium lactate mixture, etc.) and acetate buffers (e.g. acetic acid-sodium acetate mixture, acetic acid-sodium hydroxide mixture). Additionally, phosphate buffers, histidine buffers and trimethylamine salts, such as Tris can be used.

Preservatives can be added to retard microbial growth, and can be added in amounts ranging from 0.2%-1% (w/v). Suitable preservatives include phenol, benzyl alcohol, meta-cresol, methyl paraben, propyl paraben, octadecyldimethylbenzyl ammonium chloride, benzalconium halides (e.g. chloride, bromide, and iodide), hexamethonium chloride, and alkyl parabens, such as methyl or propyl paraben, catechol, resorcinol, cyclohexanol, and 3-pentanol. Isotonicifiers sometimes known as "stabilizers" can be added to ensure isotonicity of liquid compositions and include polhydric sugar alcohols, preferably trihydric or higher sugar alcohols, such as glycerin, erythritol, arabitol, xylitol, sorbitol and mannitol. Stabilizers refer to a broad category of excipients, which can range in function from a bulking agent to an additive, which solubilizes the therapeutic agent or helps to prevent denaturation or adherence to the container wall. Typical stabilizers can be polyhydric sugar alcohols (enumerated above); amino acids, such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols, such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (e.g. peptides of 10 residues or fewer); proteins, such as human serum albumin, bovine serum albumin, gelatin or immunoglobulins; hydrophylic polymers, such as polyvinylpyrrolidone monosaccharides, such as xylose, mannose, fructose, glucose; disaccharides, such as lactose, maltose, sucrose and trisaccharides, such as raffinose; and polysaccharides, such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, etc.), polyoxamers (184, 188, etc.), pluronic polyols, polyoxyethylene sorbitan monoethers (TWEEN®-20, TWEEN®-80, etc.). Non-ionic surfactants can be present in a range of about 0.05 mg/ml to about 1.0 mg/ml, or in a range of about 0.07 mg/ml to about 0.2 mg/ml.

Additional miscellaneous excipients include bulking agents (e.g. starch), chelating agents (e.g. EDTA), antioxidants (e.g. ascorbic acid, methionine, vitamin E), and co-solvents.

The formulation herein can also contain a second therapeutic agent in addition to a GPV inhibitor, e.g. an anti-GPV antibody. Examples of suitable second therapeutic agents are provided below.

The dosing schedule can vary from once a month to daily depending on a number of clinical factors, including the type of disease, severity of disease, and the patient's sensitivity to the GPV inhibitor, e.g. an anti-GPV antibody. In specific embodiments, a GPV inhibitor, e.g. an anti-GPV antibody, is administered daily, twice weekly, three times a week, every 5 days, every 10 days, every two weeks, every three weeks, every four weeks or once a month, or in any range between any two of the foregoing values, for example from every four days to every month, from every 10 days to every two weeks, or from two to three times a week, etc. The dosage of a GPV inhibitor, e.g. an anti-GPV antibody, to be administered will vary according to the particular antibody, the subject, and the nature and severity of the disease, the physical condition of the subject, the therapeutic regimen (e.g. whether a second therapeutic agent is used), and the selected route of administration; the appropriate dosage can be readily determined by a person skilled in the art.

It will be recognized by one of skill in the art that the optimal quantity and spacing of individual dosages of a GPV inhibitor, e.g. an anti-GPV antibody, will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop, the amount and/or frequency of the dosage can be altered or reduced, in accordance with normal clinical practice.

*Combination Therapy*

Preferably, the patient being treated with the GPV inhibitor, e.g. an anti-GPV antibody, is also treated with conventional coagulants. For example, a patient suffering from excessive bleeding is typically also being treated with an antifibrinolytic agent, a platelet concentrate, a coagulation factor concentrate and/or fresh frozen plasma.

Yet another aspect of the invention is the use of an inhibitor (preferably an antibody) as defined hereinabove for promoting hemostasis.

Yet another aspect of the invention is a compound (preferably an antibody) as defined hereinabove for use in reducing the bleeding time in a patient suffering from excessive bleeding.

The invention further relates to a method of reducing the bleeding time, comprising administering to a subject an effective amount of an inhibitor (preferably an antibody) as defined hereinabove.

A further aspect of this invention is a method of treating a hemorrhagic condition, comprising administering to a patient in need thereof an effective amount of an inhibitor (preferably an antibody) as defined hereinabove. The hemorrhagic condition is preferably one of the conditions described above.

A further aspect of this invention is a method of preventing a hemorrhagic condition, comprising administering to a patient in need thereof an effective amount of an inhibitor (preferably an antibody) as defined hereinabove. The hemorrhagic condition is preferably one of the conditions described above.

Overview of the nucleotide and amino acid sequences:

| SEQ ID NO: | Description |
|---|---|
| 1 | Nucleic acid sequence encoding human GPV |
| 2 | Amino acid sequence encoded by SEQ ID NO:1, i.e. human GPV including signal peptide |

(continued)

| SEQ ID NO: | Description |
|---|---|
| 3 | Amino acid sequence of human GPV lacking signal peptide |
| 4 | Nucleic acid sequence encoding murine GPV |
| 5 | Amino acid sequence encoded by SEQ ID NO:4, i.e. murine GPV including signal peptide |
| 6 | Amino acid sequence of murine GPV lacking signal peptide |

## EXAMPLES

### Results

89F12 is a monoclonal rat anti-mouse GPV antibody. It was generated by fusion of immortalized AG14 myeloma cells and spleen cells of rats, which were immunized with mouse platelets. 89F12 binds to wildtype, but not to GPV-deficient mouse platelets (Figure 1A) and 89F12 binds soluble GPV which is formed upon thrombin-cleavage of the receptor (Figure 1B). Moreover, 89F12 binds to the recombinantly expressed extracellular domain of murine GPV (Figure 1 B).

*In vivo,* 89F12-IgG or 89F12-Fab fragments have no effect on the platelet count and have no influence on the thrombin-mediated cleavage of GPV (Figure 2 and data not shown). Furthermore, 89F12 does not affect platelet activation *in vitro* as assessed by flow cytometry (Figure 2D), aggregometry or flow adhesion assays (not shown).

The effect of the anti-GPV antibody on hemostasis and thrombus formation was tested *in vivo.* Intravenous injection of 100 μg 89F12 in WT mice resulted in an accelerated time to cessation of the blood loss in the tail bleeding time assay. Simultaneous depletion of GPVI and CLEC-2 by JAQ1- and INU1-injection, respectively, resulted in a pronounced hemostatic defect since 5 of 14 mice could not stop the bleeding within the observation period of 20 min. Blockade of the extracellular domain of GPV by 89F12 could compensate for the hemostatic defect of GPVI/CLEC-2 double-depleted mice resulting in tail bleeding times in these mice comparable to 89F12-treated WT mice. This means that 89F12 can restore hemostasis in GPVI/CLEC-2 double-depleted mice. Thereby, 89F12 prevents hemostatic complications in the absence of GPVI and CLEC-2 (Figure 3). To test whether receptor dimerization or the Fc portion of the antibody are required for its effects on thrombus formation GPVI-depleted α2-deficient *(Itga2$^{-/-}$)* mice were treated with vehicle or 89F12-Fab fragments. Vehicle-treated GPVI-depleted *Itga2$^{-/-}$* mice bled more than 20 min, while 89F12-Fab fragments restored the hemostatic function of these mice (Figure 4).

Since GPV blockade promotes hemostasis, thrombus formation was also studied in 89F12-treated mice. 89F12-treated mice displayed an accelerated time to thrombus formation *in vivo.* Depletion of the principal platelet activating collagen receptor GPVI using JAQ1 led to a delayed thrombus formation time in WT mice and only 6 out of 13 vessels occluded within the observation period. Even in the absence of GPVI, 89F12-mediated blockade of GPV resulted in an earlier beginning of thrombus formation (data not shown) finally leading to a faster vessel occlusion even if compared to untreated WT controls (P<0.001, Figure 5). 89F12-mediated blockade of GPV could fully compensate for the lack of GPVI in *in vivo* thrombus formation and hemostasis.

The fact that GPV-blockade could counterbalance the absence of the (hem)ITAM receptors GPVI and CLEC-2 suggests that GPV serves as a general negative regulator of platelet activation. Therefore, we investigated whether GPV blockade could compensate for the absence of other critical signaling molecules.

Platelet activation upon vessel wall injury leads to cytoskeletal rearrangements which are crucial for conversion from a discoid to a spheric platelet shape, for granule secretion and spreading. The Rho family of small GTPases, such as RhoA, is thought to be involved in many of these processes. RhoA plays a central role in the organization of the actin cytoskeleton through the formation of stress fibers, the regulation of actomyosin contractility and in the regulation of microtubule dynamics (Bustelo XR, et al. BioEssays. 2007; 29: 356-70). Furthermore, RhoA is involved in different cellular processes downstream of Gq- and $G_{13}$-coupled agonist receptors (Pleines I, et al. Blood. 2012; 119: 1054-63).

Megakaryocyte- and platelet specific RhoA-deficient mice displayed a pronounced macrothrombocytopenia with reduction of platelet counts by 50% (Pleines I, et al. Blood. 2012; 119: 1054-63). Lack of RhoA prolonged tail bleeding times (720 ± 321 s, compared to 430 ± 267 s in WT mice). In contrast, 89F12 treatment of RhoA-deficient mice could rescue the hemostatic defect of *RhoA$^{fl/fl,\ PF4\text{-}cre}$* mice resulting in shortened tail bleeding times compared to WT mice (286 ± 118 s for 89F12-treated RhoA-deficient mice, P<0.01 compared to RhoA-deficient mice, Figure 6). This indicated that antibody-mediated GPV-blockade can also compensate for the lack of critical downstream signaling molecules.

Thrombocytopenia is a critical risk factor for bleeding. To assess the role of GPV blockade under these conditions, thrombocytopenia was induced in mice by injection of two monoclonal anti-GPIbα antibodies, which deplete circulating platelets in mice independently of immune effector mechanisms (Nieswandt B, et al. Blood. 2000; 96: 2520-7; Bergmeier W, et al. Blood. 2000; 95: 886-93). Initial platelet counts were assessed as described above for each mouse and considered 100%. Subsequent counts were assessed 1 h after injection of the antibody and were normalized to the initial values. Wildtype mice with platelet count reduction below 15% of normal displayed prolonged bleeding times or were unable to stop the bleeding within the observation period of 20 min. In contrast, platelet count reductions up to 5% of normal did not alter tail bleeding times compared to 89F12-treated mice having a normal platelet count, but showed significantly shortened tail bleeding times when compared to platelet-depleted WT-mice indicating that the blockade of GPV lowers the platelet count required to maintain normal hemostasis (not shown).

## Materials and Methods

### Mice

C57BL/6JRj (Janvier Labs) were used as wildtype control mice. Mice lacking GPV (*Gp5*$^{-/-}$), the $\alpha$2-integrin subunit (*Itga2*$^{-/-}$) were described previously (Kahn M. et al., Blood 1999. 94: 4112-21; Holtkötter O. et al., J Biol Chem. 2002. 277(13): 10789-94). Mice lacking RhoA in megakaryocytes/platelets (*RhoA*$^{fl/fl,\ Pf4\text{-}cre+/-}$) were described previously (Pleines I. et al., Blood 2012. 119: 1054-63). GPVI was depleted by injecting 100 $\mu$g of the anti-GPVI antibody, JAQ1 (Nieswandt B, et al. J Exp Med. 2001; 193: 459-69.), i.p. 6 d before the experiment. CLEC-2 was depleted by i.p. injection of 200 $\mu$g INU1 ((May F, et al. Blood. 2009; 114: 3464-72); anti-CLEC-2 antibody). GPV blockade was achieved by injecting 50 $\mu$g 89F12-IgG or Fab-fragments 30 min to 12 h before the experiment. Animal studies were approved by the district government of Lower Franconia (Bezirksregierung Unterfranken).

### Monoclonal Antibodies

### Generation of anti-GPV antibodies

Female Wistar rats, 6 to 8 weeks of age, were immunized repeatedly with mouse platelets. The rat spleen cells were then fused with mouse myeloma cells (Ag14.653) and hybridomas were selected in HAT medium. Hybridomas secreting monoclonal antibodies (mAbs) directed against platelet surface antigens were identified by flow cytometry (see below). *Gp5*$^{-/-}$ platelets were used as controls to test antibody specificity against GPV. Positive hybridomas were subcloned twice before large-scale production.

The further antibodies used are summarized in the following table.

| Antibody | Internal Name | Antigen | Described in |
|---|---|---|---|
| INU1 | 11E9 | CLEC-2 | [7] |
| JAQ1 | 98A3 | GPVI | [31] |
| p0p/B | 57E12 | GPIb | [33] |
| JON/A | 4H5 | GPIIb/IIIa | Bergmeier et al., Cytometry 2002 |

### Buffers and Media

All buffers were prepared with double-distilled water.

| Phosphate buffered saline (PBS), pH 7.14 | |
|---|---|
| NaCl | 137 mM |
| KCl | 2.7 mM |
| $KH_2PO_4$ | 1.5 mM |
| $Na_2HPO_4$ | 8 mM |

### *In vitro* Platelet Analyses

### Platelet Preparation and Washing

Mice were bled under isoflurane anesthesia from the retroorbital plexus. 700 $\mu$l blood were collected into a 1.5 ml reaction tube containg 300 $\mu$l heparin in TBS (20 U/ml, pH 7.3). Blood was centrifuged at 800 rpm (52 g; in a Eppendorf Centrifuge 5415C) for 5 min at RT. Supernatant and buffy coat were transferred into a new tube and centrifuged at 800 rpm for 6 min at RT to obtain *platelet rich plasma* (PRP). To prepare washed platelets, PRP was centrifuged at 2800 rpm (639 g) for 5 min at RT in the presence of *prostacyclin* ($PGI_2$) (0.1 μg/ml) and the pellet was resuspended in 1 ml $Ca^{2+}$-free Tyrode's buffer containing $PGI_2$ (0.1 μg/ml) and apyrase (0.02 U/ml). After 10 min incubation at 37°C the sample was centrifuged at 2800 rpm for 5 min. After resuspending the platelets once more in 1 ml $Ca^{2+}$-free Tyrode's buffer, the platelet levels were determined taking a 1:10 dilution of the platelet solution and measuring platelet counts in a Sysmex counter (see below). The pellet was resuspended in the volume of Tyrode's buffer containing apyrase (0.02 U/ml) required to obtain 500,000 platelets/μl and left to incubate for at least 30 min at 37°C before analysis.

For determination of platelet count and size, 50 μl blood were drawn from the retroorbital plexus of anesthetized mice using heparinized microcapillaries and collected into a 1.5 ml reaction tube containing 300 μl heparin in TBS (20 U/ml, pH 7.3). The heparinized blood was diluted with PBS and platelet counts and size were determined using a Sysmex KX-21N automated hematology analyzer (Sysmex Corp., Kobe, Japan).

**Flow Cytometry**

For determination of glycoprotein expression levels, platelets ($1*10^6$) were stained for 10 min at RT with saturating amounts of fluorophore-conjugated antibodies described above and analyzed directly after addition of 500 μl PBS. The reaction was stopped by addition of 500 μl PBS and samples were analyzed on a FACSCalibur (Becton Dickinson, Heidelberg, Germany). For platelet activation studies, washed blood was incubated with the indicated agonists in the presence of JON/A-PE and anti-P-selectin-FITC for 15 min.

**ELISA**

Washed platelets were prepared as described above and stimulated with 0.1 U/ml thrombin for 15 min at 37°C. Afterwards, the platelet suspension was centrifuged for 5 min at 2800 rpm, the supernatant was transferred to a new reaction tube and once again centrifuged for 5 min at maximal speed. 100 μl of the thrombin-stimulated platelet supernatant were transferred to a 96-well plate which was previously coated with 30 μg 89H11 (anti-GPV antibody) and blocked with 5% BSA/PBS. After an incubation of 1 h, the 96-well plate was washed and incubated with HRP-labelled 89F12, a second anti-GPV antibody, to detect the cleaved GPV. The HRP substrate was developed using TMB, the reaction was stopped with $H_2SO_4$ and developed in an ELISA reader at 405 nm.

***In vivo* Analyses of Platelet Function**

**Platelet depletion**

Initial platelet counts were assessed as described above for each mouse. These values were considered 100% and subsequent counts were normalized to these values. Mice were injected intravenously with 0.2 μg anti-GPIb antibodies per g mouse to induce an Fc-independent thrombocytopenia. Mice were injected 1 h before the experiment to achieve a remaining platelet count of 5-10% to induce FcγR-independent thrombocytopenia. 1 h after injection, platelet counts were again assessed.

**Mechanical Injury of the Abdominal Aorta**

To open the abdominal cavity of anesthetized mice (10-16 weeks of age), a longitudinal midline incision was performed and the abdominal aorta was exposed. A Doppler ultrasonic flow probe (Transonic Systems, Maastricht, Netherlands) was placed around the aorta and thrombosis was induced by mechanical injury with a single firm compression (15 s) of a forceps upstream of the flow probe. Blood flow was monitored until complete occlusion occurred or 30 min had elapsed.

**Bleeding Time Assay**

Mice were anesthetized by intraperitoneal injection of triple anesthesia and a 2-mm segment of the tail tip was removed with a scalpel. Tail bleeding was monitored by gently absorbing blood with filter paper at 20 s intervals without directly contacting the wound site. When no blood was observed on the paper, bleeding was determined to have ceased. The experiment was manually stopped after 20 min by cauterization.

**Statistical Analysis**

Results are shown as mean ± SD from at least three individual experiments per group. When applicable Fisher's exact test was used for statistical analysis. Otherwise, the Welch's *t* test was performed for statistical analysis. P-values <0.05 were considered statistically significant.

## Claims

1. An inhibitor of glycoprotein V (GPV) for use as a coagulant.

2. An inhibitor of glycoprotein V (GPV) for use in the treatment or prevention of a hemorrhagic condition.

3. The inhibitor for use according to claim 2, wherein said hemorrhagic condition is caused by a platelet disorder.

4. The inhibitor for use according to claim 3, wherein said platelet disorder is **characterized by** a decreased number of platelets.

5. The inhibitor for use according to any one of claims 2 to 4, wherein said hemorrhagic condition is selected from the group consisting of inflammatory bleeding, hemorrhagic stroke, excessive bleeding due to sepsis, excessive bleeding due to thrombocytopenia, excessive bleeding due to disseminated intravascular coagulation (DIC), excessive bleeding due to chemotherapy, excessive bleeding due to hemolytic-uremic syndrome, and excessive bleeding due to HIV infection.

**6.** The inhibitor for use according to any one of the preceding claims, wherein said GPV is human GPV.

**7.** The inhibitor for use according to any one of the preceding claims, wherein said inhibitor is an antibody directed against the extracellular domain of GPV, or a functional fragment or derivative of an antibody, said fragment or derivative being capable of binding to the extracellular domain of GPV.

**8.** The inhibitor for use according to claim 7, wherein said antibody is a monoclonal antibody or a functional fragment or functional derivative thereof.

**9.** The inhibitor for use according to any one of the preceding claims, wherein said inhibitor is a nucleic acid capable of reducing expression of *GP5* mRNA.

**10.** The inhibitor for use according to any one of the preceding claims, wherein said inhibitor, upon administration to a subject, does not affect the number of platelets in said subject.

**11.** The inhibitor for use according to any one of claims 2 to 10, wherein said inhibitor is used as a coagulant.

**12.** The inhibitor for use according to any one of claims 2 to 11, wherein said treatment or prevention comprises administering to a subject, preferably to a human, a pharmaceutically effective amount of said inhibitor.

**13.** The inhibitor for use according to claim 12, wherein said treatment or prevention further comprises administering to said subject a coagulant other than said inhibitor.

**14.** The inhibitor for use according to claim 13, wherein said coagulant other than said inhibitor is selected from the group consisting of an anti-fibrinolytic agent, a platelet concentrate, a coagulation factor concentrate and fresh frozen plasma.

**15.** A pharmaceutical composition comprising an inhibitor as defined in any one of the preceding claims, and a pharmaceutically acceptable excipient.

**16.** A pharmaceutical composition as defined in claim 15 for use in in the treatment or prevention of a hemorrhagic condition.

**17.** A method of treating a hemorrhagic condition in a subject, preferably a human, comprising administering to the subject an effective amount of an inhibitor as defined in any one of claims 1 to 11, or the pharmaceutical composition of claim 15.

Figure 1

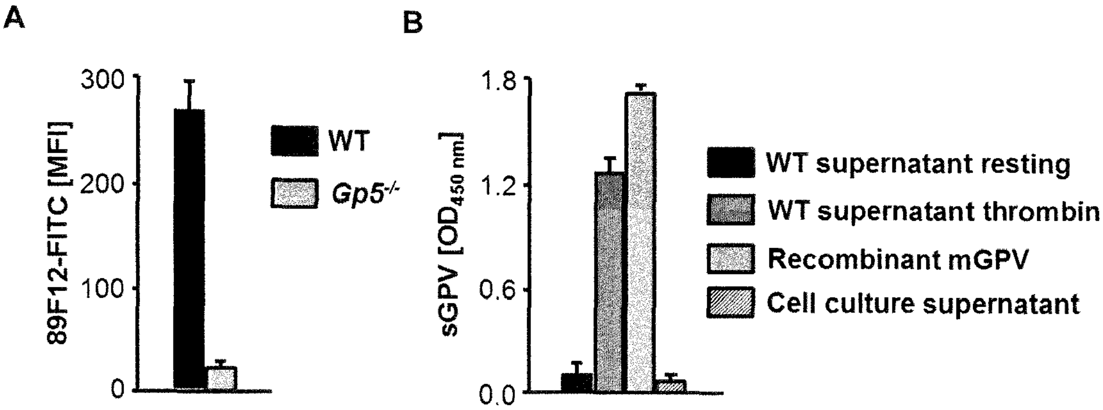
A
300
200
100
0
89F12-FITC [MFI]
WT
Gp5-/-
B
1.8
1.2
0.6
0.0
sGPV [OD450 nm]
WT supernatant resting
WT supernatant thrombin
Recombinant mGPV
Cell culture supernatant

Figure 2

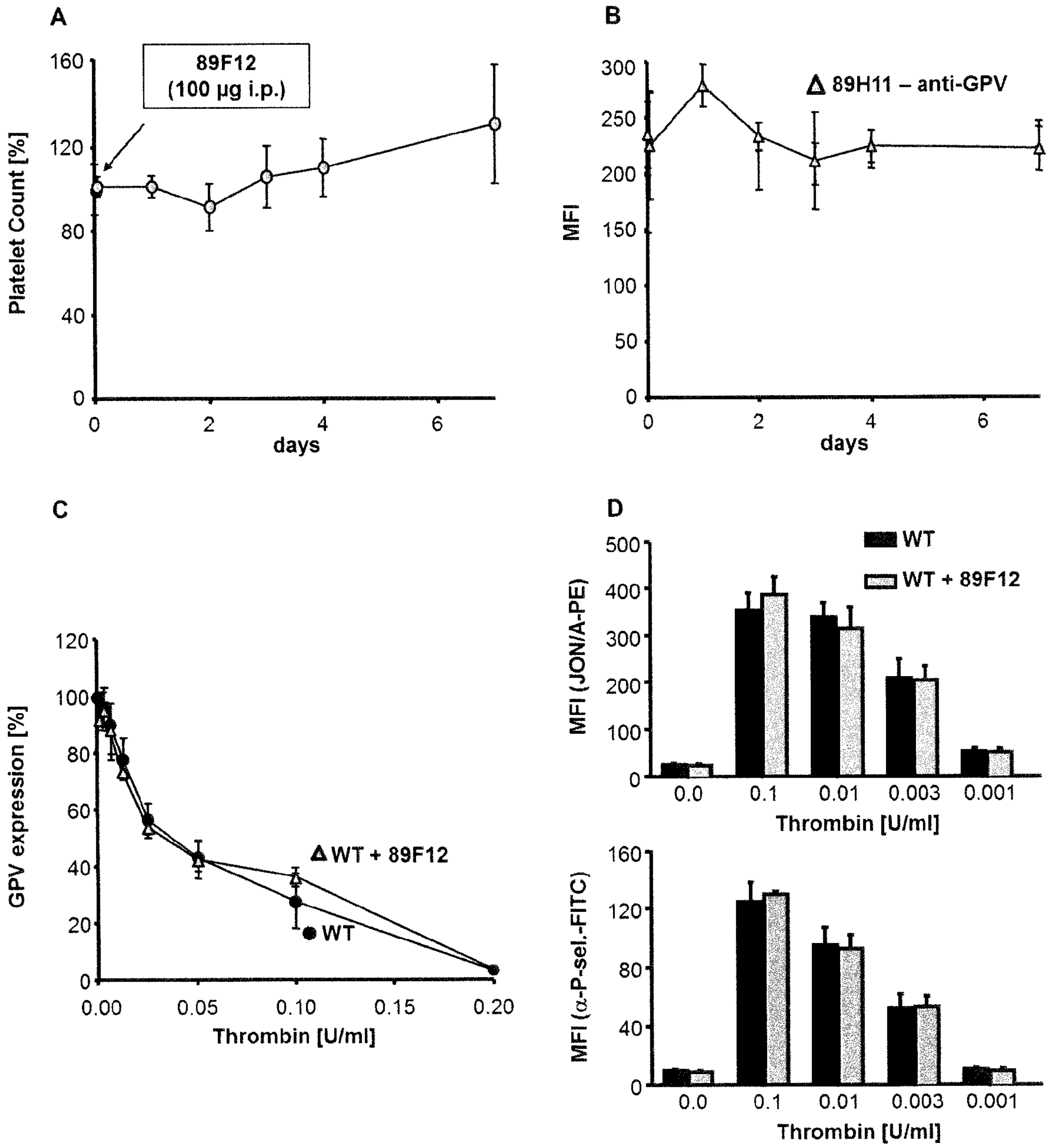
A
89F12
(100 µg i.p.)
Platelet Count [%]
days
B
Δ 89H11 – anti-GPV
MFI
days
C
GPV expression [%]
Δ WT + 89F12
● WT
Thrombin [U/ml]
D
WT
WT + 89F12
MFI (JON/A-PE)
Thrombin [U/ml]
MFI (α-P-sel.-FITC)
Thrombin [U/ml]

Figure 3

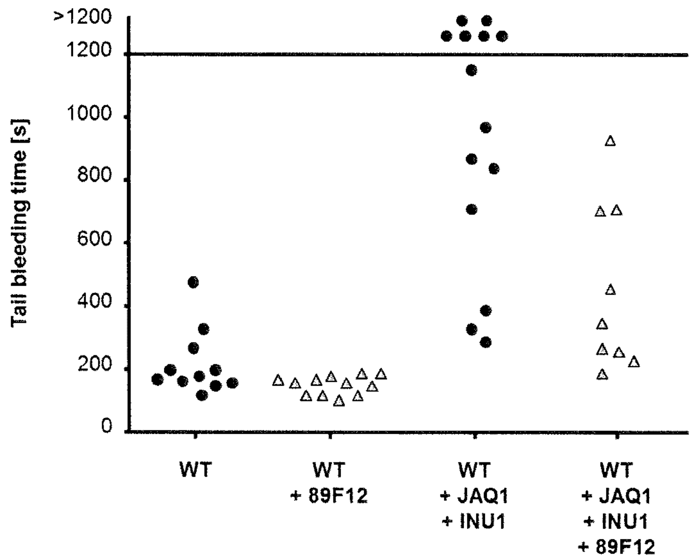


Figure 4

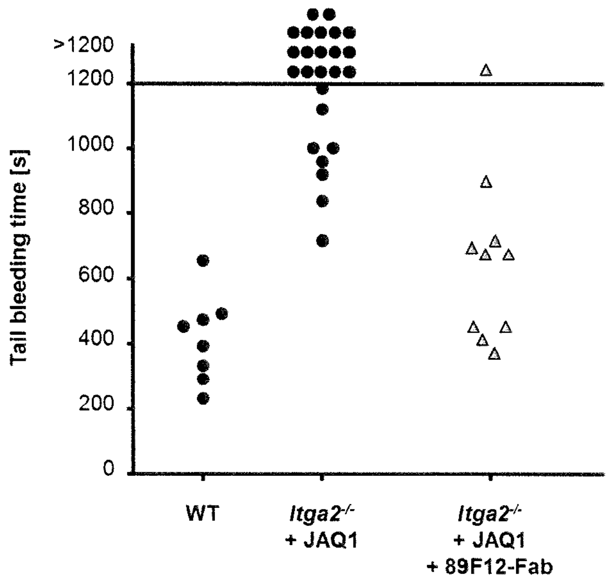

Figure 5

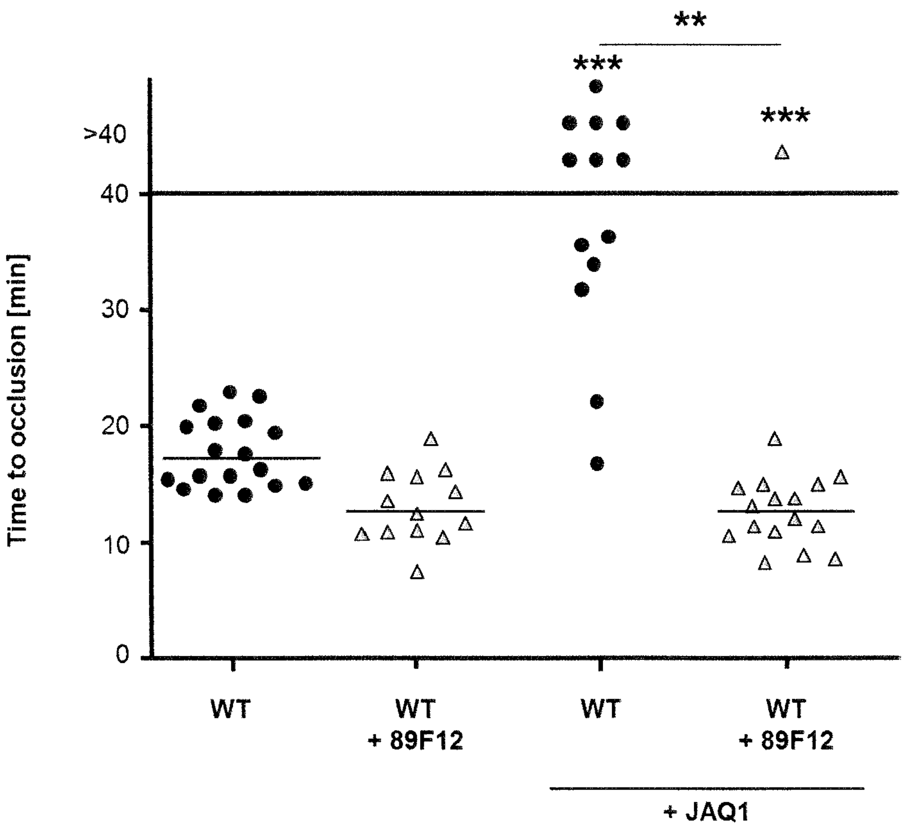


Figure 6

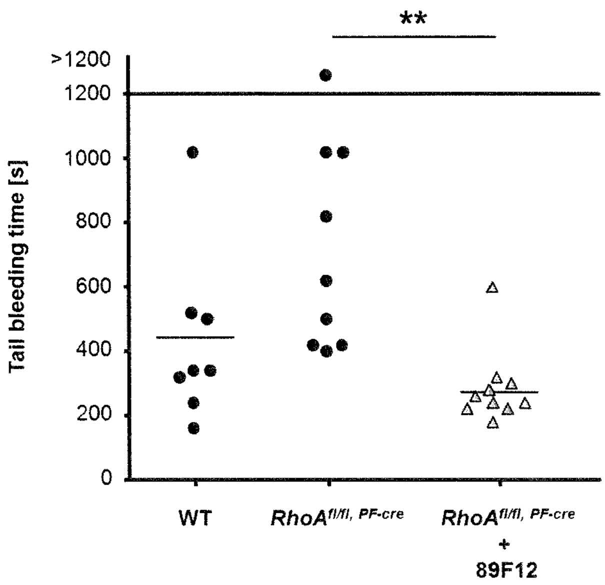

Europäisches Patentamt
European Patent Office
Office européen des brevets

# EUROPEAN SEARCH REPORT

Application Number
EP 15 20 2582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | H. NI ET AL: "Increased thrombogenesis and embolus formation in mice lacking glycoprotein V", BLOOD, vol. 98, no. 2, 15 July 2001 (2001-07-15), pages 368-373, XP055254460, US ISSN: 0006-4971, DOI: 10.1182/blood.V98.2.368 * abstract figures * | 1-17 | INV. C07K16/28 C07K16/36 A61P7/04 A61K39/395 |
| Y | RAMAKRISHNAN V ET AL: "INCREASED THROMBIN RESPONSIVENESS IN PLATELETS FROM MICE LACKING GLYCOPROTEIN V", PROCEEDINGS OF THE NATIONAL ACADEMY OF SCIENCES, NATIONAL ACADEMY OF SCIENCES, US, vol. 96, no. 23, 1 January 1999 (1999-01-01), pages 13336-13341, XP000869655, ISSN: 0027-8424, DOI: 10.1073/PNAS.96.23.13336 * abstract figures * | 1-17 | |
| X | S. MOOG: "Platelet glycoprotein V binds to collagen and participates in platelet adhesion and aggregation", BLOOD, vol. 98, no. 4, 15 August 2001 (2001-08-15), pages 1038-1046, XP055048230, ISSN: 0006-4971, DOI: 10.1182/blood.V98.4.1038 | 15 | TECHNICAL FIELDS SEARCHED (IPC): C07K A61K |
| Y | * abstract pages 1038-139 figures * | 1-14,16, 17 | |
| | -/-- | | |

The present search report has been drawn up for all claims

| Place of search | Date of completion of the search | Examiner |
|---|---|---|
| Munich | 18 April 2016 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 15 20 2582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AZORSA D O ET AL: "Measurement of GPV released by activated platelets using a sensitive immunocapture ELISA--its use to follow platelet storage in transfusion", THROMBOSIS AND HAEMOSTASIS, SCHATTAUER GMBH, DE, vol. 81, no. 1, 1 January 1999 (1999-01-01), pages 131-138, XP002754916, ISSN: 0340-6245 * abstract pages 132-133 * ----- | 15 | |
| A | KINCHINGTON P R ET AL: "THE GLYCOPROTEIN PRODUCTS OF VARICELLA-ZOSTER VIRUS GENE 14 AND THEIR DEFECTIVE ACCUMULATION IN A VACCINE STRAIN (OKA)", JOURNAL OF VIROLOGY, THE AMERICAN SOCIETY FOR MICROBIOLOGY, US, vol. 64, no. 9, 1 July 1990 (1990-07-01), pages 4540-4548, XP009000474, ISSN: 0022-538X * abstract * ----- | 1-17 | TECHNICAL FIELDS SEARCHED (IPC) |
| A | WO 95/02054 A2 (COR THERAPEUTICS INC [US]) 19 January 1995 (1995-01-19) * pages 3, 24-30 examples claims * ----- -/-- | 1-17 | |

2

The present search report has been drawn up for all claims

| Place of search | Date of completion of the search | Examiner |
|---|---|---|
| Munich | 18 April 2016 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 15 20 2582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NORIHIDE SATO ET AL: "Characterization of Monoclonal Antibodies Against Mouse and Rat Platelet Glycoprotein V (CD42d)", HYBRIDOMA, vol. 19, no. 6, 1 December 2000 (2000-12-01), pages 455-461, XP055265469, US ISSN: 0272-457X, DOI: 10.1089/027245700750053940 * abstract figures * | 1-17 | |
| A | LANZA F ET AL: "CLONING AND CHARACTERIZATION OF THE GENE ENCODING THE HUMAN PLATELET GLYCOPROTEIN V", JOURNAL OF BIOLOGICAL CHEMISTRY, AMERICAN SOCIETY FOR BIOCHEMISTRY AND MOLECULAR BIOLOGY, US, vol. 268, no. 28, 5 October 1993 (1993-10-05), pages 20801-20807, XP000867216, ISSN: 0021-9258 * abstract figures * | 1-17 | TECHNICAL FIELDS SEARCHED (IPC) |
| A | KUNISHIMA SHINJI ET AL: "Further characterization of anti-platelet monoclonal antibody HPL5 as anti-glycoprotein V antibody", ACTA HAEMATOLOGICA, S. KARGER, BASEL, CH, vol. 115, no. 1-2, 1 January 2006 (2006-01-01), pages 128-130, XP009189540, ISSN: 0001-5792 * 128-129 * | 1-17 | |

The present search report has been drawn up for all claims

| Place of search | Date of completion of the search | Examiner |
|---|---|---|
| Munich | 18 April 2016 | Bernhardt, Wiebke |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 3 of 3

## ANNEX TO THE EUROPEAN SEARCH REPORT ON EUROPEAN PATENT APPLICATION NO. EP 15 20 2582

This annex lists the patent family members relating to the patent documents cited in the above-mentioned European search report. The members are as contained in the European Patent Office EDP file on
The European Patent Office is in no way liable for these particulars which are merely given for the purpose of information.

18-04-2016

| Patent document cited in search report | Publication date | Patent family member(s) | Publication date |
|---|---|---|---|
| WO 9502054 A2 | 19-01-1995 | AU 7325794 A | 06-02-1995 |
| | | CA 2166872 A1 | 19-01-1995 |
| | | EP 0707648 A1 | 24-04-1996 |
| | | JP H09500017 A | 07-01-1997 |
| | | SG 70981 A1 | 21-03-2000 |
| | | WO 9502054 A2 | 19-01-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5807715 A
- US 4816567 A
- US 4816397 A
- US 5530101 A, Queen
- US 5585089 A
- US 5693761 A
- US 5693762 A
- US 6180370 B
- EP 239400 A
- WO 9109967 A
- US 5225539 A
- EP 0592106 A
- EP 0519596 A
- US 5565332 A
- US 4444887 A
- US 4716111 A
- WO 9846645 A
- WO 9850433 A
- WO 9824893 A
- WO 9816654 A
- WO 9634096 A
- WO 9633735 A
- WO 9110741 A
- WO 9201047 A
- US 5413923 A
- US 5625126 A
- US 5633425 A
- US 5569825 A
- US 5661016 A
- US 5545806 A
- US 5814318 A
- US 5885793 A
- US 5916771 A
- US 5939598 A
- US 5658570 A
- US 5681722 A
- US 5693780 A
- WO 2005123780 A
- US 7217797 B
- US 7361740 B
- US 7365168 B
- US 7217798 B
- US 20070280931 A
- WO 8601533 A
- EP 0392745 A
- WO 2005117984 A
- US 5219996 A
- EP 0948544 A


### Non-patent literature cited in the description


- **COUGHLIN SR.** *Nature,* 2000, vol. 407, 258-64
- **SHAPIRO MJ et al.** *JBC.,* 2000, vol. 275, 25216-21
- **NIESWANDT B et al.** *Blood,* 2003, vol. 102, 449-61
- **OFFERMANNS S.** *Circulation research.,* 2006, vol. 99, 1293-304
- **NAKANISHI-MATSUI M et al.** *Nature,* 2000, vol. 404, 609-13
- **CUNNINGHAM MA et al.** *J Exp Med,* 2000, vol. 191, 455-62
- **LUO S.-Z. et al.** *Blood,* 2007, vol. 109 (2), 603-9
- **NIESWANDT B et al.** *J Thromb Haemost.,* 2009, vol. 7, 206-9
- **VARGA-SZABO D et al.** *J Thromb Haemost.,* 2009, vol. 7, 1057-66
- **CANOBBIO I et al.** *Cellular signalling,* 2004, vol. 16, 1329-44
- **RAMAKRISHNAN V et al.** *PNAS,* 1999, vol. 96, 13336-41
- **KAHN ML et al.** *Blood,* 1999, vol. 94, 4112-21
- **DONG J-F et al.** *J Biol Chem.,* 1998, vol. 273, 31449-54
- **RAVANAT C et al.** *Blood,* 1997, vol. 89, 3253-62
- **AZORSA DO et al.** *Thrombosis and Haemostasis,* 1999, vol. 81, 131-8
- **KAHN ML et al.** *Nature,* 1998, vol. 394, 690-4
- **NI H et al.** *Blood,* 2001, vol. 98, 368-73
- **MOOG S et al.** *Blood,* 2001, vol. 98, 1038-46
- **NONNE C et al.** *J Thromb Haemost,* 2008, vol. 6, 210-2
- **TATUSOVA et al.** *FEMS Microbiol. Lett.,* 1999, vol. 174, 247-250
- **WAHL et al.** *J. Nucl. Med.,* 1983, vol. 24, 316
- **HARLOW ; LANE.** Antibodies, A Laboratory Manual. CSH Press, 1988

- **MORRISON.** *Science,* 1985, vol. 229 (4719), 1202-7
- **OI et al.** *BioTechniques,* 1986, vol. 4, 214-221
- **GILLIES et al.** *J. Immunol. Methods,* 1985, vol. 125, 191-202
- **RIECHMANN et al.** *Nature,* 1988, vol. 332, 323-7
- **PADLAN.** *Mol Immunol.,* 1991, vol. 28, 489-498
- **STUDNICKA et al.** *Prot Eng.,* 1994, vol. 7, 805-814
- **ROGUSKA et al.** *PNAS,* 1994, vol. 91, 969-973
- **JESPERS et al.** *Biotechnology,* 1988, vol. 12, 899-903
- **CANFIELD ; MORRISON.** *J Exp Med.,* 1991, vol. 173, 1483-1491
- **LUND et al.** *J Immunol.,* 1991, vol. 147, 2657-2662
- **HELLSTROM et al.** Controlled Drag Delivery. 1987, 623-53
- **THORPE et al.** *Immunol Rev.,* 1982, vol. 62, 119-58
- **DUBOWCHIK et al.** *Pharmacology and Therapeutics,* 1999, vol. 83, 67-123
- Poly(ethyleneglycol) Chemistry, Biotechnical and Biomedical Applications. Plenum Press, 1992
- Poly(ethyleneglycol) Chemistry and Biological Applications. American Chemical Society, 1997
- Bioconjugation Protein Coupling Techniques for the Biomedical Sciences. Grove Publishers, 1998
- **CHAPMAN.** *Advanced Drug Delivery Reviews,* 2002, vol. 54, 531-545
- siRNA Design: Methods and Protocols. 2012
- De novo Molecular Design. 2013
- The Aptamer Handbook: Functional Oligonucleotides and Their Applications. 2006
- **FROKJAER et al.** Pharmaceutical Formulation Development of Peptides and Proteins. Taylor & Francis, 2000
- **KIBBE et al.** Handbook of Pharmaceutical Excipients. Pharmaceutical Press, 2000
- **A. GENNARO.** Remington: The Science and Practice of Pharmacy. Lippincott, Williams, & Wilkins, 2000
- Pharmaceutical Dosage Forms and Drug Delivery Systems. Lippincott, Williams, & Wilkins, 1999
- Handbook of Pharmaceutical Excipients. Amer. Pharmaceutical Assoc, 2000
- Remington’s Pharmaceutical Sciences. 1980
- **BUSTELO XR et al.** *BioEssays,* 2007, vol. 29
- **PLEINES I et al.** *Blood,* 2012, vol. 119, 1054-63
- **NIESWANDT B et al.** *Blood,* 2000, vol. 96, 2520-7
- **BERGMEIER W et al.** *Blood,* 2000, vol. 95, 886-93
- **KAHN M. et al.** *Blood,* 1999, vol. 94, 4112-21
- **HOLTKÖTTER O. et al.** *J Biol Chem.,* 2002, vol. 277 (13), 10789-94
- **PLEINES I. et al.** *Blood,* 2012, vol. 119, 1054-63
- **NIESWANDT B et al.** *J Exp Med.,* 2001, vol. 193, 459-69
- **MAY F et al.** *Blood,* 2009, vol. 114, 3464-72
- **BERGMEIER et al.** *Cytometry,* 2002